# EUROPÄISCHE PATENTSCHRIFT

(11) **EP 2 470 488 B1**
(45) Veröffentlichungstag und Bekanntmachung des Hinweises auf die Patenterteilung: **17.02.2016**
(21) Anmeldenummer: 10745536.2
(22) Anmeldetag: 12.08.2010
(51) Int. Cl.: C07C 29/04, C07C 29/10, C07C 31/20, C07D 301/06

(54) **VERFAHREN UND VORRICHTUNG ZUR HERSTELLUNG VON ALKYLENOXIDEN UND VON ALKYLENGLYKOLEN**
METHOD AND DEVICE FOR PRODUCING ALKYLENE OXIDES AND ALKYLENE GLYCOLS
PROCÉDÉ ET DISPOSITIF DE PRODUCTION D'OXYDES D'ALKYLÈNE ET DE GLYCOLS D'ALKYLÈNE

(30) Priorität: 24.08.2009 DE 102009038398
(43) Veröffentlichungstag der Anmeldung: 04.07.2012
(73) Patentinhaber: ThyssenKrupp Industrial Solutions AG, 45143 Essen (DE)
(72) Erfinder: REIF, Ferdinand, Rudolf, 74374 Zaberfeld (DE)
(74) Vertreter: Kutzenberger, Helga
(86) Internationale Anmeldenummer: PCT/EP2010/004933
(87) Internationale Veröffentlichungsnummer: WO 2011/023300

(56) Entgegenhaltungen:
- WO-A1-02/088102
- WO-A1-2004/009568

## Beschreibung

Die vorliegende Erfindung betrifft ein Verfahren zur kombinierten Herstellung von Alkylenoxiden und von Alkylenglykolen bei gleichzeitiger Rückgewinnung der bei der Alkylenoxidherstellung als Nebenprodukte gebildeten Alkylenglykole sowie eine daran angepasste Anlage. Alternativ kann das Verfahren so durchgeführt werden bzw. kann die Anlage so betrieben werden, dass anstelle von Alkylenglykolen hauptsächlich Alkylenglykolether entstehen.

Die großtechnische Herstellung von Alkylenoxiden und von Alkylenglykolen ist allgemein bekannt.

Alkylenoxide werden großtechnisch üblicherweise durch Oxidation von Alkenen gewonnen. In den bekannten technisch relevanten Verfahren bilden sich die entsprechenden Alkylenglykole als Nebenprodukte durch Umsetzung mit im Reaktionssystem vorhandenem Wasser nach der exothermen Reaktion
Alkylenoxid + H₂O 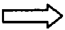 Alkylenglykol (1).

Zudem finden Folgereaktionen zu höheren Alkylenglykolen statt, wie den Di- und Trialkylenglykolen, gemäß den Formeln
Alkylenoxid + Alkylenglykol 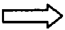 Dialkylenglykol (2), und
Alkylenoxid + Dialkylenglykol 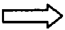 Trialkylenglykol (3).

In der Regel ergeben sich dabei Alkylenglykolkonzentrationen von bis zu 10 %. Diese Alkylenglykole werden üblicherweise als Schwersieder zusammen mit dem Wasser aus der Alkylenoxidanlage entfernt. Vor Abgabe in die Umwelt muss dieses Prozesswasser gereinigt werden, typischerweise in einer biologischen Reinigungsanlage. Die in diesem Prozesswasser vorhandenen Alkylenglykole verursachen einen sehr hohen CSB-Wert, bzw. eine sehr hohe Fracht an biologisch abbaubaren Stoffen, so dass die biologische Reinigungsanlage sehr groß dimensionert werden muss und das Wasser in der Regel vorher verdünnt werden muss. Zudem stellen Alkylenglykole Wertstoffe dar. Alternativ zur vergrößerten Biologie können die Alkylenglykole destillativ entfernt werden, was jedoch eines hohen energetischen und apparativen Aufwands bedarf.

Alkylenglykole werden großtechnisch durch Umsetzung von reinen Alkylenoxiden in wässriger Phase entsprechend Formeln (1), (2) und (3) gewonnen. Die Reaktionen nach (1), (2) und (3) finden unter einem erheblichen Wasserüberschuss von beispielsweise etwa 10-20-fach überstöchiometrisch statt. Dieses Wasser wird in einem ersten Schritt aus dem Reaktionssystem in der Regel durch eine mehrstufige Verdampfung entfernt und nach Kondensation erneut in den Reaktor zurückgeführt. Das entstandene Alkylenglykolgemisch wird dann in einer Serie von Rektifikationskolonnen in die einzelnen Glykole aufgetrennt. Durch die großen Mengen des zu verdampfenden Wassers ergeben sich große zuzuführende Energiemengen.

Erfindungsgemäß werden eine herkömmliche und bekannte Alkylenoxidanlage und eine herkömmliche und bekannte Alkylenglykolanlage derart modifiziert und miteinander kombiniert, dass
- sowohl Alkylenoxid als auch Alkylenglykole bzw. alternativ dazu Alkylenglykolether produziert werden können,
- die in der Alkylenoxidanlage gebildeten Alkylenglykole und Alkylenglykolether als Wertstoffe zurückgewonnen werden und somit auch die nachgeschaltete Reinigungsanlage entlastet wird,
- eine Energieintegration stattfinden kann,
- kein Frischwasser zugeführt werden muss, und
- nicht oder nur teilweise gereinigtes Alkylenoxid verwendet werden kann.

Außerdem erfolgt die Herstellung von Alkylenoxiden häufig in Methanol als Lösungsmittel. Dieses muss nach der Umsetzung zurückgewonnen werden, um wieder in den Prozess rückgeführt werden zu können. Ein solches Aufbereitungsverfahren wird in DE 102 33 388 A1 (entsprechend WO 2004/009566 A1) beschrieben. Weitere Herstellungsverfahren von Alkylenoxiden verwenden andere organische Lösungsmittel. Aus WO 2009/001948 A1 ist die Umsetzung von Propylen, Wasserstoff und Sauerstoff zu Propylenoxid in Acetonitril oder in wässrigem Acetonitril beschrieben.

In konventionellen Verfahren zur Herstellung von Alkylenoxiden wird das Ausgangsmaterial Alken in flüssiger Phase mit einem Oxidationsmittel umgesetzt. Die Reaktion kann unter Zugabe eines Katalysators durchgeführt werden. Als Oxidationsmittel haben sich Chlor, Hydroperoxide und bevorzugt Peroxide, besonders bevorzugt Wasserstoffperoxid, bewährt. Die Reaktion findet in einem Reaktor statt, typischerweise in einem Rohrbündelreaktor. Darin reagiert das Alken mit dem Oxidationsmittel zum Alkylenoxid, gegebenenfalls unter Ausbildung von Zwischenprodukten, wie Chlorhydrinen, die anschließend zum Alkylenoxid umgesetzt werden. Neben dem gewünschten Produkt Alkylenoxid entstehen bei der Umsetzung in geringem Maße Alkylenglykole. Diese Verbindungen stellen einerseits wertvolle Chemikalien dar, sind aber aufgrund der geringen Menge in der Regel nur mit hohem apparativen Aufwand und mit hohem Energieaufwand gewinnbar, so dass diese bislang bei der Aufarbeitung der Reaktionsrückstände beseitigt werden mussten. DE 102 33 382 A1 beschreibt ein Verfahren zur kontinuierlich betriebenen Reindestillation des bei der koppelproduktfreien Synthese von Propylenoxid anfallenden 1,2-Propylenglykols. US 7,332,634 B2 beschreibt ein kontinuierliches Verfahren zur Abtrennung von 1,2-Propylenglykol, das als Nebenprodukt bei der Herstellung von Propylenoxid anfällt.

Nach dem Reaktor für die Herstellung des Alkylenoxids erfolgt die Auftrennung des Reaktionsgemisches in Produkt, in nicht umgesetzte Edukte, Wasser und gegebenenfalls vorhandene organische Lösemittel, typischerweise in Rektifikationskolonnen. Leicht siedende Nebenprodukte verlassen die Anlage vorzugsweise mit dem Spülgas, schwer siedende Nebenprodukte verlassen die Anlage vorzugsweise mit dem zur Verdünnung der Reaktanden eingesetzten Wasser bzw. mit dem bei der Reaktion gebildeten Wasser. Dieses Abwasser muss in der Regel vor dem Einleiten in eine biologische Abwasserbehandlung weiter verdünnt werden. Ein alternativer Ansatz trennt aus dem unverdünnten Abwasser destillativ organische Verbindungen ab, so dass schwächer belastetes Abwasser der folgenden Behandlungsstufe zugeführt wird. Jedoch bedarf es hier eines hohen energetischen Aufwandes.

Beispiele für die Herstellung von Alkylenglykolen aus Alkylenoxiden sind in der WO 2004/085375 A1, EP 0 226 799 B1, US-A-3,574,772, US-A-4,937,393, DE 29 38 115 C2 und DE 197 26 508 A1 zu finden. Es ist auch möglich, Alkylenglykole durch direkte Umsetzung aus Alkenen zu erzeugen. Beispiele dafür finden sich in US-A-4,203,926 sowie in US-A-4,308,409.

In einem konventionellen Verfahren zur Herstellung von Alkylenglykolen wird das Ausgangsmaterial Alkylenoxid mit Wasser vermischt und durch einen Reaktor geleitet, typischerweise einen einfachen adiabaten Rohrreaktor. Darin reagiert das Alkylenoxid mit Wasser in exothermer Reaktion zum Alkylenglykyol. Neben dem einfachen Alkylenglykol werden in der Regel höhere Alkylenglykole gebildet, also hauptsächlich Dialkylenglykol und Trialkylenglykol sowie in sehr geringen Anteilen gegebenenfalls noch höhere Alkylenglykole. Diese Verbindungen stellen ebenfalls wertvolle Chemikalien dar. Typische Verhältnisse von Alkylenglykol zu Di- und Trialkylenglykol betragen etwa 100:10:1. Die Reaktion kann unter Zugabe eines Katalysators durchgeführt werden. Das Verfahren arbeitet mit großen Mengen an Wasser, welche in der Regel im Kreis gefahren werden. Diese sind erforderlich, um die Reaktionswärme abzuführen und um durch Verdünnung des Alkylenoxids und des Alkylenglykols die Bildung von höheren Alkylenglykolen zu unterdrücken.

Nachdem das Reaktionsgemisch den Reaktor verlassen hat, erfolgt zunächst die Abtrennung des Wassers, beispielsweise in einer Rektifikationskolonne oder in einer einfachen Verdampfung. Zwecks Energieeinsparung werden häufig mehrere Verdampfer oder Rektifikationskolonnen miteinander verschaltet.

Nach der Wasserabtrennung erfolgt eine Auftrennung in die verschiedenen Alkylenglykole. Das wird in der Regel in Rektifikationskolonnen durchgeführt. Dabei erfolgt der Reihe nach die Abtrennung des Alkylenglykols, des Dialkylenglykols und schließlich des Trialkylenglykols jeweils über Kopf oder über Seitenabzug. Vorhandene Schwersieder werden im Sumpf der Trialkylenglykol-Kolonne abgezogen und in der Regel verworfen, beispielsweise durch Verbrennung. In einigen Verfahrensvarianten wird wegen der geringen Menge des gebildeten Trialkylenglykols auf die dritte Kolonne verzichtet und bereits der Sumpf der Dialkylenglykol-Kolonne aus der Anlage ausgeschleust und verworfen. Dieses Verfahren bzw. diese Verfahrensvarianten sind allgemein bekannt.

Anlagen zur Herstellung von Alkylenoxiden und zur Herstellung von Alkylenglykolen wurden bislang separat betrieben, obwohl derzeit etwa 20 % des produzierten Alkylenoxids für die Herstellung von Alkylenglykolen verwendet wird.

Es ist allerdings bereits vorgeschlagen worden, Alkylenglykole und Alkylenoxid in einer Anlage herzustellen und diese Produkte anschließend voneinander zu trennen. Ein Beispiel dafür findet sich in der WO 02/088102 A1. Allerdings werden hier nicht-flüssige und nicht-wässrige Systeme verwendet und die Reaktionen finden in der Gasphase statt. Das Verfahren hat bis heute keine großtechnische Umsetzung erfahren.

Es sind bereits Kombinationen von Anlagen bekannt, in denen verschiedene Stoffe miteinander umgesetzt und aufgetrennt werden. DE 10 2004 054 047 A1 beschreibt ein Verfahren zur Herstellung von 1,6-Hexandiol aus einem Adipinsäure, 6-Hydroxycarbonsäure und 1,4-Cyclohexandiole enthaltenden Carbonsäuregemisch durch Veresterung des Carbonsäuregemischs, destillativer Abtrennung der 1,4-Cyclohexandiole, Hydrierung der gereinigten Esterfraktion und destillativer Gewinnung des 1,6-Hexandiols. DE 10 2008 007 081 A1 beschreibt ein Verfahren zur Herstellung von n-Buten-Oligomeren und 1-Buten aus technischen Mischungen von C4-Kohlenwasserstoffen. Dabei wird ein Ausgangsprodukt zunächst gereinigt und destillativ aufgearbeitet. Anschließend wird eine gewonnene Schwersiederfraktion katalytisch umgesetzt, wobei die darin enthaltenen n-Butene oligomerisiert werden. DE 10 2005 006 974 A1 beschreibt ein kontinuierliches Verfahren zur Herstellung von Cyclohexyl(meth)acrylat. Dabei wird Cyclohexanol mit Rein(meth)acrylsäure sauer verestert, neutralisiert, gewaschen und anschließend durch mehrstufige Destillation gereinigt. In diesen Verfahren bzw. Anlagen werden weder Alkylenoxid noch Alkylenglykole erzeugt.

Auch die Koppelung einer Anlage zur Herstellung von Alkylenoxid mit einer Anlage zur Herstellung von Alkylenglykol ist bereits vorgeschlagen worden. Ein Beispiel dafür findet sich in DE 102 33 385 A1 (entsprechend WO 2004/009568 A1). Hier wird das in den beiden Anlagenteilen gebildet Alkylenglykol aus dem jeweiligen Anlagenteil abgeführt und bei der Aufarbeitung der Alkylenglykole kombiniert. Außerdem wird dem Reaktor zur Herstellung von Alkylenglykol frisches Wasser zugeführt. Das in diesen Dokumenten beschriebene Verfahren beinhaltet eine Kopplung der Erzeugung von Propylenoxid mit der Erzeugung von Propylenglykolen. Allerdings wird das aus der Propylenoxidanlage stammende Rohpropylenoxid im zweiten Reaktor mit Wasser umgesetzt, welches aber nicht dem ersten Reaktor entstammt. Außerdem werden im vorbekannten Verfahren die in den Stufen der Propylenoxiderzeugung und der Propylenglykolerzeugung erhaltenen Propylenglykolgemische vereinigt und danach werden die einzelnen Propylenglykole destillativ abgetrennt. Beim vorbekannten Verfahren wird also das aus dem ersten Reaktor stammende von Propylen und gegebenenfalls von Propylenoxid befreite Reaktionsgemisch am zweiten Reaktor vorbei geschleust und später mit dem aus dem zweiten Reaktor stammenden Propylenglykolgemisch vereinigt. Dieses Reaktionsgemisch aus dem ersten Reaktor enthält erhebliche Mengen an Wasser, das grundsätzlich entweder vor oder nach der Vereinigung mit dem aus dem zweiten Reaktor stammenden Propylenglykolgemisch entfernt werden muss. Somit verlangt das vorbekannte Verfahren eine separate Abtrennung des Wassers aus dem ersten Reaktor und des Wassers aus dem zweiten Reaktor, welches einen erheblichen Aufwand an Energie und Investitionskosten zur Folge hat, da dann eine energieintensive Kreislaufführung des Wassers erfolgen muss.

Es wird ständig nach Verfahren und Maßnahmen gesucht, mit denen die Effizienz dieser Verfahren verbessert werden kann und mit denen diese Grundchemikalien ökonomischer hergestellt werden können.

Aufgabe der vorliegenden Erfindung ist die Bereitstellung eines einfachen und energieeffizienten Verfahrens zur kombinierten Herstellung von Alkylenoxiden und von Alkylenglykolen und gegebenenfalls von Alkylenglykolether und zur Rückgewinnung der in der Alkylenoxidanlage in Nebenreaktionen gebildeten Alkylenglykole und Alkylenglykolether sowie einer dafür geeigneten Anlage.

Die vorliegende Erfindung betrifft ein Verfahren zur Herstellung von Alkylenoxid und von Alkylenglykolen in einer integrierten Anlage mit einer ersten Teilanlage zur Herstellung von Alkylenoxid durch Umsetzung von C₂-C₆-Olefin mit einem Oxidationsmittel in flüssiger und Wasser enthaltender Phase, wobei Alkylenglykole und gegebenenfalls Alkylenglykolether als Nebenprodukte entstehen, und mit der ersten Teilanlage verbunden eine zweite Teilanlage zur Herstellung von Alkylenglykolen durch Umsetzung von Alkylenoxid mit Wasser in flüssiger Phase, wobei das aus der ersten Teilanlage stammende und zumindest Wasser und Alkylenglykole sowie gegebenenfalls Alkylenglykolether aufweisende Reaktionsgemisch, aus der ersten Teilanlage in die zweite Teilanlage eingeleitet wird.

Beim erfindungsgemäßen Verfahren wird der alkylenglykolhaltige und gegebenenfalls alkylenglykoletherhaltige Prozesswasserstrom aus der Alkylenoxidanlage gegebenenfalls nach dem Einstellen eines bestimmten pH-Wertes direkt in den Reaktor der Alkylenenglykolanlage geleitet und nach dessen beispielsweise destillativer Abtrennung aus der Alkylenglykolanlage ausgeleitet. Bei geeigneter Wahl der Kapazität kann die energieintensive Wasserrückführung entfallen. Befinden sich im Abwasser weitere werthaltige Komponenten, so kann das Abwasser vor der Einleitung in die Abwasserbehandlung einer weiteren Behandlung unterzogen werden. Die entwässerten Glykole werden bevorzugt destillativ in das Mono-, Di- und Trialkylenglykol und gegebenenfalls in höherwertige Alkylenglykole aufgetrennt. Da oftmals Alkylenoxidanlagen und Alkylenglykolanlagen am selben Standort betrieben werden, eignet sich die Erfindung auch zur Umrüstung und Kombination bestehender Anlagen.

Aus dem aus der ersten Teilanlage stammenden und zumindest Wasser, Alkylenglykole und Alkylenglykolether aufweisenden Reaktionsgemisch werden vorzugsweise vor dem Einleiten in die zweite Teilanlage ein oder mehrere Komponenten abgetrennt, vorzugsweise das olefinische Ausgangsmaterial, das Reaktionsprodukt Alkylenoxid und gegebenenfalls das organische Lösungsmittel.

In einer alternativen Ausführungsform kann dass erfindungsgemäße Verfahren so betrieben werden, dass in der zweiten Teilanlage hauptsächlich Alkylenglykolether entstehen. Dazu wird die Umsetzung zum Alkylenoxid in der ersten Teilanlage in wässrig-alkoholischer Lösung durchgeführt und aus dem aus der ersten Teilanlage stammenden und zumindest Wasser, Alkohol, Alkylenglykole und Alkylenglykolether aufweisenden Reaktionsgemisch werden vorzugsweise vor dem Einleiten in die zweite Teilanlage ein oder mehrere Komponenten abgetrennt, vorzugsweise das olefinische Ausgangsmaterial und gegebenenfalls das Reaktionsprodukt Alkylenoxid. Durch das Einleiten von größeren Mengen Alkohol in die zweite Teilanlage werden die dort vorhandenen bzw. gebildeten Alkylenglykole verethert bzw. das Alkylenoxid wird direkt mit Alkohol zu Alkylenglykolen umgesetzt, so dass als Hauptprodukte die entsprechenden Mono- oder Diether der Alkylenglykole entstehen.

Mit dem erfindungsgemäßen Verfahren kann eine aufwändige Aufarbeitung des aus der ersten Teilanlage stammenden wässrigen Reaktionsgemisches entfallen, da dieses direkt in die zweite Teilanlage eingeleitet wird. Eine Aufarbeitung des im Gesamtverfahren eingesetzten bzw. erzeugten Wassers und gegebenenfalls vorhandenen organischen Lösungsmittels kann für die erste Teilanlage entfallen und vollständig nach Durchlaufen der zweiten Teilanlage vorgenommen werden. Dieses ermöglicht eine erhebliche Einsparung an Energie und an Investitionskosten.

Das aus der zweiten Teilanlage ausgeschleuste Wasser und das gegebenenfalls in Resten vorhandene organische Lösungsmittel wird vorzugsweise einer Abwasserbehandlung zugeführt. Das Wasser kann aber auch teilweise im Kreis gefahren werden und zumindest anteilig zusammen mit dem Wasser aus der ersten Teilanlage in die zweite Teilanlage eingespeist werden. Eine Rückführung des aus der zweiten Teilanlage stammenden Wassers ist besonders dann besonders bevorzugt, wenn der Betrieb der ersten Teilanlage unterbrochen oder verringert werden muss. Auch dass aus der zweiten Teilanlage abgeführte organische Lösungsmittel kann nach einer Abtrennung ganz oder teilweise im Kreis gefahren werden und zumindest anteilig zusammen mit frischem organischen Lösungsmittel in die erste Teilanlage eingespeist werden.

Die aus der ersten Teilanlage stammenden Alkylenglykole werden in der zweiten Teilanlage mit den in dieser erzeugten Alkylenglykole kombiniert und in der zweiten Teilanlage aufgearbeitet, vorzugsweise durch Rektifikation.

Die aus der ersten Teilanlage stammenden Alkylenglykolether werden in der zweiten Teilanlage mit den in dieser erzeugten Alkylenglykolethern kombiniert und nach Verlassen der zweiten Teilanlage entweder mit dem Wasser abgeführt und gegebenenfalls nach der Abtrennung von organischem Lösungsmittel sodann verworfen oder diese Alkylenglykolether werden als Wertstoff nach Verlassen der zweiten Teilanlage vom Wasser und dem gegebenenfalls vorhandenen organischen Lösungsmittel abgetrennt, und danach aufgearbeitet, vorzugsweise durch Rektifikation.

Wird die Reaktion in der zweiten Teilanlage so geführt, dass aus den dort vorliegenden bzw. gebildeten Alkylenglykolen hauptsächlich Alkylenglykolether entstehen, so werden die aus der ersten Teilanlage stammenden Alkylenglykolether in der zweiten Teilanlage mit den in dieser erzeugten Alkylenglykolethern kombiniert und in der zweiten Teilanlage aufgearbeitet, vorzugsweise durch Rektifikation. Die vorliegende Erfindung betrifft ein Verfahren zur Herstellung von Alkylenoxid und von Alkylenglykolen in einer integrierten Anlage mit einer ersten Teilanlage zur Herstellung von Alkylenoxid, die mindestens einen Alkylenoxidreaktor R1 sowie eine nachgeschaltete Trennvorrichtung A und gegebenenfalls eine daran nachgeschaltete Trennvorrichtung B, welche hauptsächlich aus Rektifikationskolonnen bestehen, aufweist und mit der ersten Teilanlage verbunden eine zweite Teilanlage zur Herstellung von Alkylenglykolen, die mindestens einen Alkylenglykolreaktor R2 sowie mindestens eine nachgeschaltete Trennvorrichtung C und daran nachgeschaltet mindestens eine Trennvorrichtung D aufweist, wobei das Verfahren folgende Schritte aufweist:
i) Erzeugen von Alkylenoxid durch Umsetzung von C₂-C₆-Olefin mit einem Oxidationsmittel in flüssiger Phase in der ersten Teilanlage,
ii) Auftrennen des den Alkylenoxidreaktor R1 verlassenden Reaktionsgemisches in der Trennvorrichtung A in einen Teilstrom a enthaltend im wesentlichen C₂-C₆-Olefin und gegebenenfalls weitere mit diesem abgetrennte Bestandteile des Reaktionsgemisches und in einen Teilstrom b enthaltend Wasser, Alkylenoxid, Alkylenglykol und weitere Komponenten des Reaktionsgemisches sowie gegebenenfalls organisches Lösungsmittel,
iii) gegebenenfalls Auftrennen des Teilstromes b in der Trennvorrichtung B in einen Teilstrom c enthaltend im wesentlichen Alkylenoxid und gegebenenfalls weitere mit diesem abgetrennte Bestandteile des Reaktionsgemisches und in einen Teilstrom d enthaltend Wasser, Alkylenglykol und weitere Komponenten des Reaktionsgemisches sowie gegebenenfalls organisches Lösungsmittel,
iv) Erzeugen von Alkylenglykol und von höheren Alkylenglykolen durch Umsetzung von Alkylenoxid mit Wasser in wässriger Phase in der zweiten Teilanlage, indem
v) der Teilstrom b aus der Trennvorrichtung A und/oder der Teilstrom d aus der Trennvorrichtung B, gegebenenfalls nach einer Einstellung des pH-Werts, in den Alkylenglykolreaktor R2 geleitet wird,
vi) Alkylenoxid aus Teilstrom c und/oder aus anderern Quellen in den Alkylenglykolreaktor R2 geleitet wird, wobei dieser Schritt im Falle des Einleitens des Teilstromes b aus der Trennvorrichtung A in den Alkylenglykolreaktor R2 entfallen kann,
vii) das den Alkylenglykolreaktor R2 verlassende und Alkylenglykole und gegebenenfalls Alkylenglykolether enthaltende Reaktionsgemisch in der Trennvorrichtung C in einen Teilstrom e bestehend im wesentlichen aus dem im Reaktionsgemisch enthaltenen Wasser und gegebenenfalls weiteren mit dem Wasser abgetrennten Bestandteilen des Reaktionsgemisches sowie gegebenenfalls zusätzlich in einen Teilstrom f bestehend im wesentlichen aus dem im Reaktionsgemisch enthaltenen organischen Lösungsmittel und gegebenenfalls weiteren mit dem organischen Lösungsmittel abgetrennten Bestandteilen des Reaktionsgemisches und in einen Teilstrom g gebildet aus den Alkylenglykolen und den übrigen nicht in den Teilstrom e und gegebenenfalls nicht in den Teilstrom f übergegangenen Teilen des Reaktionsgemisches sowie gegebenenfalls in einen Teilstrom i enthaltend Salze und andere Feststoffe aus dem Reaktionsgemisch und gegebenenfalls in einen Teilstrom j enthaltend Monoalkylenglykolether aufgetrennt wird,
viii) zumindest ein Teil des Teilstromes e aus der Anlage ausgeschleust wird und vorzugsweise in eine Abwasserreinigungsanlage verbracht wird und der gegebenenfalls verbliebene Teils des Teilstromes e in den Eingang des Alkylenglykolreaktors R2 rückgeführt wird,
ix) der gegebenenfalls vorliegende Teilstrom f gegebenenfalls nach weiterer Aufarbeitung in den Alkylenoxidreaktor R1 zurückgeführt wird, und
x) die im Teilstrom g enthaltenen Alkylenglykole in der Trennvorrichtung D aufgetrennt werden.

Das erfindungsgemäße Verfahren wird in einer Kombination zweier Anlagen betrieben und gestattet eine erheblich einfachere Behandlung des in den Anlagen entstehenden Prozesswassers. Auch verringert sich die Menge des Prozesswassers, da durch die Reaktion des Alkylenoxids zu Alkylenglykolen Wasser verbraucht wird.

Vorteilhafterweise wird, abgesehen von einer optionalen pH-Werteinstellung, keine Behandlung des aus der ersten Teilanlage stammenden Prozesswassers benötigt, da dieses ganz oder zum überwiegenden Teil in der zweiten Teilanlage eingesetzt wird. Das erfindungsgemäße Verfahren zeichnet sich gegenüber herkömmlichen Verfahren dadurch aus, dass die in der ersten Teilanlage als Nebenprodukte entstandenen Alkylenglykole die zweite Teilanlage durchlaufen und als Wertstoffe gewonnen werden können und dass auf einen Wasserkreislauf in der zweiten Teilanlage verzichtet werden kann bzw. dass dieser Wasserkreislauf erheblich kleiner dimensioniert werden kann. Prozesswasser aus der Alkylenoxid-Anlage wird in der zweiten Teilanlage einfach zuerst mit Alkylenoxid gemischt und anschließend direkt dem Alkylenglykol-Reaktor zugeführt. Der Kreislauf des Wassers wird unterbrochen bzw. stark verringert. Das von Alkylenglykol befreite Prozesswasser wird nicht oder nur zum geringen Teil rückgeführt, sondern der Abwasserbiologie zugeführt und muss nicht weiter mit Wasser verdünnt werden. Daher kann die Abwasserbiologie kleiner als in herkömmlichen Anlagen dimensioniert werden.

Aus Gründen der Verfahrensökonomie empfiehlt es sich außerdem, damit die Alkylenglykol-Anlage unabhängig vom Prozesswasseraufkommen produzieren kann, für diese Anlage die Möglichkeit einer Wasserrückführung vorzusehen. Auch kann die erste Teilanlage nur mit der Alkylenglykolrückgewinnung in der zweiten Teilanlage betrieben werden, indem z.B. der Teilstrom d abgesperrt wird.

Der erfindungsgemäße Ansatz besitzt mehrere Vorteile: Generell ist es vorteilhaft, der Alkylenoxid-Anlage eine Alkylenglykol-Anlage nachzuschalten, da dann zumindest ein Teilstrom des produzierten Alkylenoxids noch Wasser enthalten darf. So muss das für die Herstellung von Alkylenglykol vorgesehene Alkylenoxid nicht unbedingt am Kopf einer Alkylenoxid-Reinigungskolonne abgezogen werden, sondern es kann ein Seitenabzug verwendet werden. Damit lassen sich sowohl Investitionskosten (der Teil oberhalb des Seitenabzugs kann kleiner dimensioniert werden) als auch Betriebskosten (ein hoher Rückfluss ist nur für das wasserfreie Alkylenoxid erforderlich) einsparen. Sollte anstelle des erhöhten Rückflusses ein Molekularsieb zur Feinabtrennung des Wassers verwendet werden, so muss nur der Anteil des Alkylenoxids behandelt werden, für den "Wasserfreiheit" gewünscht ist.

Weitere Vorteile sind die bereits genannte Verwertung des bei der AlkylenoxidHerstellung erzeugten Alkylenglykols, die einfachere Abwasserbehandlung, die unmittelbare Verwendung der Reaktionsenergie für das Verdampfen des Abwassers sowie die Verminderung der Abwassermenge.

In einer bevorzugten Ausführungsform des erfindungsgemäßen Verfahrens ist für das Prozessabwassergemisch aus der ersten Teilanlage keine gesonderte Abwasserbehandlung erforderlich; das Prozesswasser aus der ersten Teilanlage wird hier vollständig in die zweite Teilanlage übergeführt und wird in einer der zweiten Teilanlage nachgeschalteten Abwasserreinigungsanlage behandelt.

Die beiden Teilanlagen benötigen im Vergleich zu einzeln betriebenen Anlagen keine größeren Modifikationen. Im Vergleich mit einer isolierten Alkylenoxidanlage mit nachgeschalteter Abwasseraufbereitung wird für das erfindungsgemäße Verfahren nur ein Teil des zweiten Anlagenteils zusätzlich benötigt. Dabei handelt es sich im wesentlichen um den Reaktor für die Herstellung des Alkylenglykols sowie um die Trennvorrichtungen für die Di- und Trialkylenglykole, welche aufgrund der guten Trennbarkeit der monomeren, dimeren und trimeren Alkylenglykole und der geringen Mengenströme vergleichsweise klein dimensioniert sein können.

In der ersten Teilanlage können beliebige Verfahren zur Herstellung von Alkylenoxiden aus Alkenen und Oxidationsmitteln betrieben werden. Als großtechnische Verfahren sind das Chlorhydrinverfahren, die Prileschajew-Reaktion sowie das HPPO-Verfahren bekannt.

Beim Chlorhydrinverfahren wird Alken mit Chlor zum entsprechenden Chlorhydrin umgesetzt und in basischer wässrige Phase wird ein Teil des Chlorhydrins zu Alkylenoxid umgesetzt. Bei der Prileschajew-Reaktion wird ein Alken über die Umsetzung mit einem Hydroperoxid zum Alkylenoxid umgesetzt. Beim HPPO-Verfahren wird ein Alken mit einem Peroxid, vorzugsweise mit Wasserstoffperoxid, zum Alkylenoxid umgesetzt.

Im erfindungsgemäßen Verfahren kommen C₂-C₆-Olefine zum Einsatz. Es werden vorzugsweise alpha-Olefine eingesetzt. Beispiele dafür sind Ethylen, Propylen, alpha-Buten, alpha-Penten oder alpha-Hexen.

Besonders bevorzugt wird das erfindungsgemäße Verfahren zur Herstellung von Ethylenoxid und Ethylenglykolen, wie Ethylenglykol, Diethylenglykol und Triethylenglykol, eingesetzt.

Ganz besonders bevorzugt wird das erfindungsgemäße Verfahren zur Herstellung von Propylenoxid und Propylenglykolen, wie Propylenglykol, Dipropylenglykol und Tripropylenglykol, eingesetzt.

Die Erzeugung des Alkylenoxids erfolgt in flüssiger Phase. Dabei kommt das Oxidationsmittel vorzugsweise in wässriger oder in wässrig-alkoholischer Lösung zum Einsatz, beispielsweise als wässrige Lösung eines Peroxids, wie Wasserstoffperoxid, oder eines Hydroperoxids, wie Benzoylhydroperoxid. Die eigentliche Reaktion verläuft in der flüssigen Phase. Dabei kommen vorzugsweise Wasser oder mit Wasser mischbare organische polare Flüssigkeiten, insbesondere Alkohole in Frage. Besonders bevorzugt wird Methanol eingesetzt. Das Reaktionssystem kann in Abhängigkeit von der Konzentration einzelner Bestandteile ein- oder mehrphasig sein. So kann beispielsweise eine wässrige Lösung des Oxidationsmittels mit einem Alkohol als Lösungsmittel und mit dem Ausgangsprodukt ein einphasiges oder ein zweiphasiges Reaktionsgemisch bilden. Außerdem wird in der Regel noch ein Katalysator eingesetzt; dieser kann als Feststoff im Reaktionsgemisch suspendiert sein und/oder in Form eines Feststoffbettes angeordnet sein.

In einer bevorzugten Variante des erfindungsgemäßen Verfahrens wird in Schritt i) Propylen mit einem Peroxid oder Hydroperoxid in flüssiger, vorzugsweise in wässriger oder wässrig-alkoholischer Phase katalytisch zu Propylenoxid umgesetzt. Ganz besonders bevorzugt kommt dabei Wasserstoffperoxid als Oxidationsmittel zum Einsatz. Ebenfalls besonders bevorzugt wird die Umsetzung in Methanol durchgeführt.

Der erste Anlagenteil umfasst einen Alkylenoxidreaktor für die Umsetzung zum Alkylenoxid sowie eine Trennvorrichtung A zur Rückgewinnung des nicht umgesetzten Ausgangsmaterials und gegebenenfalls weiterer damit abtrennbarer Bestandteile des Reaktionsgemisches, wie organisches Lösemittel und Wasser, und gegebenenfalls eine der Trennvorrichtung A nachgeschaltete Trennvorrichtung B zur Abtrennung des gebildeten Alkylenoxids vom Wasser und vom gegebenenfalls vorhandenem organischen Lösungsmittel, wie Methanol, sowie von weiteren Bestandteilen des Reaktionsgemisches.

In der ersten Teilanlage können beliebige Alkylenoxidreaktoren eingesetzt werden. Es lassen sich ein oder mehrere solcher Reaktoren verwenden, die parallel oder hintereinander geschaltet sein können. Vorzugsweise wird in der ersten Teilanlage ein Alkylenoxidreaktor eingesetzt, ganz besonders bevorzugt zwei oder drei Alkylenoxidreaktoren, die in Serie geschaltet sind. Ist in dieser Beschreibung nachstehend von dem oder von einem Alkylenoxidreaktor die Rede, so sind darunter ein oder mehrere Alkylenoxidreaktoren in beliebiger Verschaltung zu verstehen.

In der ersten Teilanlage können beliebige Trennvorrichtungen A und gegebenenfalls B eingesetzt werden. Die Trennvorrichtungen können aus einer oder mehreren Einheiten bestehen; diese können parallel oder hintereinander geschaltet sein. Vorzugsweise werden in der ersten Teilanlage Trennvorrichtungen A und B eingesetzt, die jeweils aus mehreren Einheiten bestehen.

Bevorzugt eingesetzte Alkylenoxidreaktoren R1 sind Rohrbündelreaktoren. Bevorzugt eingesetzte Trennvorrichungen A bzw. B sind Rektifikationskolonnen. Die Trennvorrichtungen A und B bestehen bevorzugt jeweils aus einer oder aus mehreren Rektifikationskolonnen. So lässt sich in einer bevorzugten Verfahrensvariante zunächst das nicht umgesetzte Ausgangsmaterial in einer Trennvorrichtung A, die hier als Rektifikationskolonne ausgebildet ist, vom Reaktionsgemisch abtrennen. Das abgetrennte Olefin kann dabei weitere Bestandteile des Reaktionsgemisches enthalten, welche einen ähnlichen Siedepunkt wie das Olefin aufweisen bzw. ein Azeotrop mit diesem bilden. Diese Bestandteile können in einer nachgeschalteten Rektifikationskolonne oder einer anderen Reinigungsstufe vom Olefin, welches anschließend vorzugsweise in den Alkylenoxid-Herstellungsprozess zurückgeführt wird, abgetrennt werden und vorzugsweise der Verbrennung zugeführt werden.

Nach der Abtrennung des Olefins aus dem Reaktionsgemisch wird letzteres anschließend vorzugsweise in einer weiteren Serie von Rektifikationskolonnen als Trennvorrichtung B weiter aufgereinigt. Alternativ kann diese Stufe entfallen und das vom Olefin befreite Reaktionsgemisch kann direkt in den Alkylenglykolreaktor eingespeist werden. Vorzugsweise wird jedoch eine Trennvorrichtung B verwendet. In einer Rektifikationskolonne wird das gebildete Alkylenoxid als Kopfprodukt und/oder als Seitenabzug abgetrennt (Teilstrom c), gegenenenfalls mit weiteren Bestandteilen des Reaktionsgemisches mit einem ähnlichen Siedepunkt bzw. mit azeotropbildendenden Bestandteilen. In einer weiteren Rektifikationskolonne wird bevorzugt ein Großteil des organischen Lösungsmittels (z.B. Methanol) vom verbliebenem Reaktionsgemisch abgetrennt und einer weiteren Destillation, z.B. in einer weiteren Rektifikationskolonne, zur Abtrennung des organischen Lösungsmittels vom Wasser und von weiteren Bestandteilen des Reaktionsgemisches unterzogen. Das so wieder gewonnene organische Lösungsmittel wird vorzugsweise in den Alkylenoxidreaktor R1 rückgeführt (Teilstrom h). Das Sumpfprodukt dieser Rektifikationskolonne weist in der Regel Wasser als Hauptbestandteil auf, sowie organisches Lösungsmittel, Salze, Katalysatorreste, mittel- und hochsiedende organische Bestandteile und bei der Alkylenoxidherstellung gebildete Alkylenglykole und gegebenenfalls Alkylenglykolether als Nebenbestandteile. Das Sumpfprodukt dieser bevorzugten Verfahrensvariante wird als Teilstrom d in den Alkylenglykolreaktor eingespeist, wobei diesem Teilstrom d in den vorangegangenen Stufen der Aufarbeitung angefallenes Wasser zugesetzt sein kann.

Bevorzugt wird mindestens ein Teil des im Alkylenoxidreaktor gebildeten Alkylenoxids in den Alkylenglykolreaktor der zweiten Teilanlage geleitet.

In einer alternativen Verfahrensvariante kann ein Teil des Teilstromes b aus der Trennvorrichtung A und/oder ein organisches Lösungsmittel direkt in den Alkylenglykolreaktor eingespeist werden und der Rest des Teilstromes b wird einer weiteren Aufarbeitung in der Trennvorrichtung B unterworfen, wobei der dabei erhaltene Teilstrom d ebenfalls in den Alkylenglykolreaktor eingeleitet werden kann.

In einer weiteren alternativen Verfahrensvariante erfolgt in der Trennvorrichtung B keine Auftrennung von Wasser und organischem Lösungsmittel, sondern diese beiden Stoffe werden gemeinsam in den Alkylenglykolreaktor eingeleitet. Diese Verfahrensvariante kommt dann bevorzugt zur Anwendung, wenn man neben Alkylenglykolen einen hohen Anteil von Alkylenglykolethern als Wertstoff anstrebt.

In einer bevorzugten Verfahrensvariante wird als Trennvorrichtung B eine Serie von Rektifikationskolonnen verwendet, wobei in einer davon ein Teil des Alkylenoxids über den Kolonnenkopf abgezogen wird, ein weiterer Teil als Seitenabzug und wobei mindestens ein Sumpfprodukt den Teilstrom d bildet.

Vorzugsweise wird zwischen dem Alkylenoxidreaktor und dem Alkylenglykolreaktor das im Alkylenoxidreaktor nicht umgesetzte Oxidationsmittel, vorzugsweise das nicht umgesetzte Peroxid, insbesondere das nicht umgesetzte Wasserstoffperoxid, durch Zugabe eines Reduktionsmittels oder eines Zersetzungskatalysators beseitigt. Dazu befindet sich zwischen dem Alkylenoxidreaktor und dem Alkylenglykolreaktor noch eine Vorrichtung zur Beseitigung von im Alkylenoxidreaktor nicht umgesetzen Oxidationsmittel. Diese Vorrichtung kann an unterschiedlichen Positionen zwischen dem Alkylenoxidreaktor und dem Alkylenglykolreaktor angebracht sein. Enthält der Teilstrom b und/oder der Teilstrom d große Mengen an organischem Lösungsmittel, wie Methanol, und wird dieses vor dem Einleiten in den Alkylenglykolreaktor aus dem betreffenden Teilstrom abgetrennt, so befindet sich die Vorrichtung zur Beseitigung von nicht umgesetzem Oxidationsmittel vor der Abtrennvorrichtung für das organische Lösungsmittel.

In der zweiten Teilanlage können beliebige Verfahren zur Herstellung von Alkylenglykolen sowie gegebenenfalls von Alkylenglykolethern aus Alkylenoxiden betrieben werden. In der Regel handelt es sich dabei um Umsetzungen von Alkylenoxid mit Wasser in neutralem, basischem oder saurem Medium. Diese Verfahren werden bevorzugt ohne den Einsatz von Katalysatoren oder unter Einsatz von sauren Katalysatoren, wie Säuren, z.B. Mineralsäuren, oder sauren Ionenaustauscherharzen, durchgeführt. Derartige Verfahren und dafür geeignete Katalysatoren sind dem Fachmann bekannt.

Wird in der zweiten Teilanlage neben der Bildung von Alkylenglykolen hauptsächlich die Weiterreaktion zu Alkylenglykolethern angestrebt, so erfolgt das in wässrigalkoholischem Medium, vorzugsweise in wässrig-methanolischem Medium mit einem Überschuss von Methanol. Die Umsetzung kann auch in diesem Falle bei sauren, neutralen oder basischen pH-Werten erfolgen.

Bevorzugt wird ein Verfahren, bei dem der Teilstrom d aus der Trennvorrichtung B und/oder der Teilstrom b aus der Trennvorrichtung A oder ein Teil davon vor dem Einleiten in den Alkylenglykolreaktor R2 mit einem Teil des Teilstromes e aus der Trennvorrichtung C, also der Abtrennvorrichtung für Wasser, kombiniert wird.

Weiterhin wird ein Verfahren bevorzugt, bei dem der Teilstrom b und/oder ein organisches Lösungsmittel in den Alkylenglykolreaktor eingeführt wird und bei dem die Auftrennung des den Alkylenglykolreaktor verlassenden und Alkylenglykole sowie gegebenenfalls Alkylenglykolether enthaltenden Reaktionsgemisches in der Trennvorrichtung C so erfolgt, dass der Teilstrom e im wesentlichen aus dem im Reaktionsgemisch enthaltenen Wasser besteht und gegebenenfalls aus Ethern, die sich aus Teilen des Alkylenoxids und anderen Bestandteilen des Reaktionsgemisches gebildet haben und bei dem der Teilstrom f im wesentlichen aus dem im Reaktionsgemisch enthaltenen organischen Lösungsmittel besteht und gegebenenfalls aus Ethern, die sich aus Teilen des Alkylenoxids und anderen Bestandteilen des Reaktionsgemisches gebildet haben.

Das zur Herstellung der Alkylenglykole erforderliche Alkylenoxid kann aus beliebigen Quellen stammen. Bevorzugt wird ein Verfahren, bei dem das in den Alkylenglykolreaktor R2 geleitete Alkylenoxid ganz oder teilweise aus der ersten Teilanlage stammt.

In der zweiten Teilanlage können beliebige Alkylenglykolreaktoren R2 eingesetzt werden. Es lassen sich ein oder mehrere solcher Reaktoren verwenden, die parallel oder hintereinander geschaltet sein können. Vorzugsweise wird in der zweiten Teilanlage ein Alkylenglykolreaktor R2 eingesetzt, ganz besonders bevorzugt zwei oder drei Alkylenglykolreaktoren R2, die in Serie geschaltet sind. Ist in dieser Beschreibung nachstehend von dem oder von einem Alkylenglykolreaktor R2 die Rede, so sind darunter ein oder mehrere Alkylenglykolreaktoren R2 in beliebiger Verschaltung zu verstehen.

In der zweiten Teilanlage können beliebige Trennvorrichtungen C und D eingesetzt werden. Die Trennvorrichtungen können aus einer oder mehreren Einheiten bestehen; diese können parallel oder hintereinander geschaltet sein. Vorzugsweise werden in der zweiten Teilanlage Trennvorrichtungen C und D eingesetzt, die jeweils aus mehreren Einheiten bestehen.

Bevorzugt eingesetzte Alkylenglykolreaktoren sind Rohrreaktoren. Bevorzugt eingesetzte Trennvorrichungen C und D sind Rektifikationskolonnen. Im Falle der Trennvorrichtungen C werden auch Dünnschichtverdampfer bevorzugt eingesetzt. Es können auch Kombinationen von Rektifikationskolonnen und Dünnschichtverdampfern eingesetzt werden.

Das aus dem Alkylenglykolreaktor ausgeschleuste Reaktionsgemisch wird anschließend in der Trennvorrichtung C vom darin enthaltenen Wasser und gegebenenfalls von darin enthaltenen Salzen und anderen Feststoffen befreit. In Abhängigkeit von der eingesetzten Trenntechnik können dabei weitere im Reaktionsgemisch enthaltene Bestandteile, wie das bei der Reaktion verwendete organische Lösungsmittel und Nebenprodukte, im abgetrennten Wasser vorhanden sein, beispielsweise bei destillativer Trennung Komponenten mit ähnlichem Siedepunkt wie Wasser oder Komponenten, die mit Wasser ein Azeotrop bilden. Wichtig ist, dass im abgetrennten Wasser keine oder möglichst wenig Anteile der Wertstoffe Alkylenglykol enthalten sind und - falls man zusätzlich die Erzeugung von Alkylenglykolethern anstrebt - keine oder möglichst wenige Anteile der Wertstoffe Alkylenglykolether enthalten sind. Die Abtrennung des Wassers aus dem aus dem Alkylenglykolreaktor ausgeschleusten Reaktionsgemisch kann destillativ erfolgen, vorzugsweise in Rektifikationskolonnnen, oder durch Membranfiltration oder mit anderen geeigneten Trenntechniken. Das abgetrennte Wasser gegebenenfalls kombiniert mit weiteren damit abgetrennten Bestandteilen des Reaktionsgemisches bildet den Teilstrom e. Die gegebenenfalls abgetrennten Salze und andere Feststoffe kombiniert mit weiteren damit abgetrennten Bestandteilen des Reaktionsgemisches bilden den Teilstrom i. Gegebenenfalls anwesende Alkylenglykolether können in dieser Stufe teilweise abgetrennt werden; es werden hauptsächlich Monoalkylenglykolether abgetrennt, welche denTeilstrom j bilden. Die höheren Alkylenglykolether fallen haupsächlich in der Alkylenglykolfraktion an, wo sie gegebenenfalls später abgetrennt werden können.

Vorzugsweise besteht die Trennvorrichtung C zur Abtrennung des Wassers vom aus dem Alkylenglykolreaktor ausgeschleusten Reaktionsgemisch aus mehreren parallel geschalteten Rektifikationskolonnen sowie vorzugsweise aus mindestens einer nachgeschalteten Rektifikationskolonne. In die parallel geschalteten Rektifikationskolonnen werden jeweils Teile des aus dem Alkylenglykolreaktor R2 ausgeschleusten Reaktionsgemisches eingeleitet. Dabei wird in einer besonders bevorzugten Ausführungsform Wärmeenergie aus dem Kopf mindestens einer Rektifikationskolonne zum Erwärmen des Sumpfes mindestens einer anderen Rektifikationskolonne genutzt und die Rektifikationskolonnen werden bei unterschiedlichen Drucken betrieben, so dass das Kopfprodukt jeweils aus Wasser und organischen Bestandteilen besteht. Alternativ kann ein Teil der Wärmeenergie für die Beheizung anderer Kolonnen in der ersten Teilanlage und/oder der zweiten Teilanlage benutzt werden, beispielsweise für die Beheizung von Kolonnen zur Auftrennung der Alkylenglykole. Die Wasser enthaltenden Sumpfprodukte aus den parallel geschalteten Rektifikationskolonnen werden vorzugsweise in die nachgeschaltete Rektifikationskolonne eingeleitet und dort wird das restliche Wasser abgetrennt.

Für den Fall, dass in den Alkylenglykolreaktor der Teilstrom b und/oder der Teilstrom d enthaltend unter anderem größere Mengen an organischem Lösungmittel eingeschleust wurden und/oder dass in den Alkylenglykolreaktor aus anderen Quellen stammendes organisches Lösungsmittel zugeführt wurde, wird in der Trennvorrichtung C vorzugsweise neben dem Wasser auch das organische Lösungsmittel aus dem Reaktionsgemisch abgetrennt. Die Abtrennung des organischen Lösungsmittels kann vor oder nach der Abtrennung des Wassers erfolgen. Auch hier können in Abhängigkeit von der eingesetzten Trenntechnik dabei weitere im Reaktionsgemisch enthaltene Bestandteile, wie Wasser oder Nebenprodukte im abgetrennten organischen Lösungsmittel vorhanden sein, beispielsweise bei destillativer Trennung Komponenten mit ähnlichem Siedepunkt wie das organische Lösungsmittel. Wichtig ist auch hier, dass im abgetrennten organischen Lösungsmittel keine oder möglichst wenig Anteile der Wertstoffe Alkylenglykol und gegebenenfalls Alkylenglykolether enthalten sind. Die Abtrennung des organischen Lösungsmittels aus dem aus dem Alkylenglykolreaktor ausgeschleusten Reaktionsgemisch kann ebenfalls destillativ erfolgen, vorzugsweise in Rektifikationskolonnnen, oder durch Membranfiltration oder mit anderen geeigneten Trenntechniken. Das abgetrennte organische Lösungsmittel bildet den Teilstrom f.

In einer bevorzugten Verfahrensvariante erfolgt die Auftrennung des den Alkylenglykolreaktor verlassenden und Alkylenglykole enthaltenden Reaktionsgemisches in der Trennvorrichtung C in solcher Weise, dass der Teilstrom e im wesentlichen aus dem im Reaktionsgemisch enthaltenen Wasser besteht und gegebenenfalls aus geringen Teilen von organischen Lösemittel und Ethern, die sich aus Teilen des Alkylenoxids und anderen Bestandteilen des Reaktionsgemisches gebildet haben.

Das als Teilstrom e abgetrennte Wasser wird entweder vollständig aus der Anlage ausgeschleust und beispielsweise einer biologischen Abwasserreinigung zugeführt oder ein Teil des abgetrennten Wassers wird wieder in den Eingang des Alkylenglykolreaktor zurückgeführt und mit dem Wasser aus dem Teilstrom b und/oder aus dem Teilstrom d, der aus dem ersten Anlagenteil stammt, vereinigt. Der gegebenenfalls abgetrennte Teilstrom i wird zur Entsorgung aus der Anlage ausgeschleust oder weiter verarbeitet. Der gegebenenfalls abgetrennte Teilstrom j wird als Wertstoff verwendet oder einer weiteren Aufarbeitung unterworfen.

In einer weiteren bevorzugten Verfahrensvariante erfolgt die Auftrennung des den Alkylenglykolreaktor verlassenden und Alkylenglykole enthaltenden Reaktionsgemisches in der Trennvorrichtung C in solcher Weise, dass ein Teilstrom e erzeugt wird sowie ein Teilstrom f, der im wesentlichen aus dem im Reaktionsgemisch enthaltenen organischen Lösungsmittel besteht und gegebenenfalls aus geringen Teilen von Wasser und Ethern, die sich aus Teilen des Alkylenoxids und anderen Bestandteilen des Reaktionsgemisches gebildet haben. Gegegebenenfalls können in dieser Trennstufe C Salze und weitere Feststoffe, z.B. Katalysatorbestandteile, abgetrennt werden, welche den Teilstrom i bilden, bzw. es können Monoalkylenglykolether abgetrennt werden, welche den Teilstrom j bilden.

In einer besonders energieeffizienten Variante des erfindungsgemäßen Verfahrens weist die Trennvorrichtung C mindestens eine Rektifikationskolonne auf, in der ein Großteil des vorhandenen Wassers, vorzugsweise 90 bis 98 % des vorhandenen Wassers, abgetrennt werden und es ist eine weitere Rektifikationskolonne vorhanden, in welcher der Rest des Wassers abgetrennt wird.

Das als Teilstrom f abgetrennte organische Lösungsmittel wird entweder direkt oder vorzugsweise nach weiterer Aufreinigung in den Alkylenoxidreaktor zurückgeführt.

Der nach Abtrennung des Teilstroms e, gegebenenfalls des Teilstroms i und/oder j und gegebenenfalls des Teilstroms f verbliebene Teilstrom g enthält Alkylenglykole und gegebenenfalls Alkylenglykolether als Wertstoffe. Dabei handelt es sich um ein Gemisch unterschiedlicher Alkylenglykole und gegebenenfalls unterschiedlicher Alkylenglykolether sowie um weitere Bestandteile des Reaktionsgemisches, beispielsweise Salze, organische Nebenprodukte und Katalysatorrückstände. Die Auftrennung der Alkylenglykole und der gegebenenfalls vorhandenen Alkylenglykolether kann nach beliebigen, dem Fachmann bekannten Verfahren erfolgen. In der Regel werden dafür Rektifikationskolonnen eingesetzt.

In einer weiteren bevorzugten Verfahrensvariante wird eine Abtrennvorrichtung für Alkylenglykol mit mehreren hintereinander geschalteten Stufen verwendet, wobei in der ersten Stufe das Alkylenglykol abgetrennt wird, in der zweiten Stufe das Dialkylenglykol und in einer gegebenenfalls vorhandenen dritten Stufe das Trialkylenglykol gegebenenfalls zusammen mit weiteren Mittel- oder Schwersiedern, und wobei das in der letzten Stufe verbliebene Sumpfprodukt aus der Anlage ausgeschleust wird.

Enthält der aufzureinigende Teilstrom g neben Alkylenglykolen größere Anteile an Alkylenglykolethern, so können diese ebenfalls in der Abtrennvorrichtung für Alkylenglykole mit mehreren hintereinander geschalteten Stufen abgetrennt werden. Bei Vorhandensein von Alkylenglykolethern können weitere Stufen vorgesehen werden. Das in der letzten Stufe verbliebene Sumpfprodukt wird aus der Anlage ausgeschleust.

Besonders bevorzugt wird ein Verfahren, bei dem eine Trennvorrichtung D für Alkylenglykol und für Alkylenglykolether verwendet wird, wobei in der ersten Stufe das Alkylenglykol und gegebenenfalls Dialkylenglykolether abgetrennt werden, in einer zweiten Stufe das Dialkylenglykol, in einer dritten Stufe Trialkylenglykolether, in einer gegebenenfalls vorhandenen vierten Stufe das Trialkylenglykol gegebenenfalls zusammen mit weiteren Mittel- oder Schwersiedern, und dass das verbliebene Sumpfprodukt aus der Anlage ausgeschleust wird.

Bei den Stufen zur Auftrennung der Alkylenglykole und gegebenenfalls der Alkylenglykolether handelt es sich bevorzugt um in Reihe geschaltete Rektifikationskolonnen.

Alternativ dazu können die Alkylenglykole in Rektifikationskolonnen mit Kopf- und Seitenabzügen aufgetrennt werden. Der Begriff Rektifikationskolonne schließt im Rahmen der vorliegenden Beschreibung auch Trennwandkolonnen mit ein.

Die Erfindung betrifft ebenfalls eine Anlage zur Herstellung von Alkylenoxid und von Alkylenglykolen und gegebenenfalls von Alkylenglykolethern mit folgenden Elementen:
A) erste Teilanlage zur Herstellung von Alkylenoxid
B) zweite Teilanlage zur Herstellung von Alkylenglykolen und gegebenenfalls von Alkylenglykolethern, wobei
   die erste Teilanlage mit der zweiten Teilanlage verbunden ist und mindestens eine Leitung aufweist, durch welche das aus der ersten Teilanlage stammende und zumindest Wasser, Alkylenglykole und Alkylenglykolether aufweisende Reaktionsgemisch aus der ersten Teilanlage in die zweite Teilanlage eingeleitet wird, sowie den Elementen C) bis F), wobei
C) die erste Teilanlage mindestens einen Alkylenoxidreaktor R1 sowie eine nachgeschaltete Trennvorrichtung A zur Auftrennung des den Alkylenoxidreaktor verlassenden Reaktionsgemisches in einen Teilstrom a enthaltend im wesentlichen C₂-C₆-Olefin und gegebenenfalls weitere mit diesem abgetrennte Bestandteile des Reaktionsgemisches und in einen Teilstrom b enthaltend Wasser, Alkylenoxid, Alkylenglykol und weitere Komponenten des Reaktionsgemisches sowie gegebenenfalls organisches Lösungsmittel, und gegebenenfalls eine der Trennvorrichtung A nachgeschaltete Trennvorrichtung B zum Auftrennen des Teilstromes b in einen Teilstrom c enthaltend im wesentlichen Alkylenoxid und gegebenenfalls weitere mit diesem abgetrennte Bestandteile des Reaktionsgemisches und in einen Teilstrom d enthaltend Wasser, Alkylenglykol und weitere Komponenten des Reaktionsgemisches sowie gegebenenfalls organisches Lösungsmittel,
D) die zweite Teilanlage mindestens einen Alkylenglykolreaktor R2 sowie mindestens eine nachgeschaltete Trennvorrichtung C und daran nachgeschaltet mindestens eine Trennvorrichtung D aufweist, wobei in der Trennvorrichtung C das den Alkylenglykolreaktor R2 verlassende Reaktionsgemisch in einen Teilstrom e bestehend im wesentlichen aus dem im Reaktionsgemisch enthaltenen Wasser und gegebenenfalls weiteren mit dem Wasser abgetrennten Bestandteilen des Reaktionsgemisches, sowie gegebenenfalls zusätzlich in einen Teilstrom f bestehend im wesentlichen aus dem im Reaktionsgemisch enthaltenen organischen Lösungsmittel und gegebenenfalls weiteren mit dem organischen Lösungsmittel abgetrennten Bestandteilen des Reaktionsgemisches und in einen Teilstrom g gebildet aus den Alkylenglykolen und den übrigen nicht in den Teilstrom e und nicht den Teilstrom f übergegangenen Teilen des Reaktionsgemisches sowie gegebenenfalls in einen Teilstrom i enthaltend Salze und andere Feststoffe aus dem Reaktionsgemisch und/oder gegebenenfalls in einen Teilstrom j enthaltend Monoalkylenglykolether aufgetrennt aufgetrennt wird,
E) der Alkylenglykolreaktor R2 mindestens eine Leitung E für die Zuführung von Alkylenoxid aufweist, wobei diese Leitung E) im Falle der Zuführung des Teilstromes b in den Alkylenglykolreaktor R2 entfallen kann, und wobei
F) mindestens eine Leitung F vorgesehen ist, durch welche zumindest ein Teil des Teilstroms b und/oder des Teilstroms d aus der ersten Teilanlage in den Alkylenglykolreaktor R2 der zweiten Teilanlage eingeleitet wird.

Die Teilanlagen sind - wie oben ausgeführt - dem Fachmann bekannt und bestehen aus bekannten Komponenten. Die Verschaltung dieser Anlagen zu einer integrierten Anlage ist bislang nicht beschrieben worden.

In einer bevorzugten Variante der erfindungsgemäßen Anlage ist zwischen dem Alkylenoxidreaktor R1 und dem Alkylenglykolreaktor R2 eine Vorrichtung vorgesehen, in der das im Alkylenoxidreaktor R1 nicht umgesetzte Oxidationsmittel beseitigt wird.

In einer bevorzugten Ausführungsform der erfindungsgemäßen Anlage ist eine Leitung G) vorgesehen, durch die zumindest ein Teil des Teilstromes e in eine Abwasserreinigungsanlage verbracht wird.

In einer weiteren bevorzugten Ausführungsform der erfindungsgemäßen Anlage ist eine Leitung H) vorgesehen, durch welche zumindest ein Teil des Teilstroms f, vorzugsweise nach weiterer Aufreinigung, in den Alkylenoxidreaktor R1 zurückgeführt wird.

In einer weiteren bevorzugten Ausführungsform der erfindungsgemäßen Anlage ist eine Leitung I) vorgesehen, durch die zumindest ein Teil des Teilstromes a in den Alkylenoxidreaktor R1 rückgeführt wird.

In einer weiteren bevorzugten Ausführungsform der erfindungsgemäßen Anlage ist eine Leitung J) vorgesehen, durch die ein Teil des Teilstromes e in den Eingang des Alkylenglykolreaktors R2 rückgeführt werden kann.

In einer weiteren bevorzugten Ausführungsform der erfindungsgemäßen Anlage ist eine Leitung K) vorgesehen, durch welche Salze und andere im Reaktionsgemisch enthaltene Feststoffe als Teilstrom i aus der Anlage ausgeschleust werden.

In einer weiteren bevorzugten Ausführungsform der erfindungsgemäßen Anlage ist eine Leitung L) vorgesehen, durch welche Monoalkylenglykolether als Teilstrom j aus der Anlage ausgeschleust wird.

Besonders bevorzugt wird eine Anlage mit Leitungen F) und J), bei der Mittel vorgesehen sind, mit der die Menge der über Leitungen F) und J) in den Alkylenglykolreaktor eingeführten Teilströme geregelt werden kann.

In einer bevorzugten Anlage handelt es sich bei der Trennvorrichtung A um mindestens einen Flash oder um mindestens einen Verdampfer, worin das Olefin gegebenenfalls zusammen mit anderen Leichtsiedern abgezogen wird und der Rückstand den Teilstrom b bildet.

In einer bevorzugten Anlage handelt es sich bei der Trennvorrichtung B um eine Rektifikationskolonne oder um eine Gruppe von miteinander verschalteten Rektifikationskolonnen, worin das Alkylenoxid über den Kolonnenkopf sowie gegebenenfalls zusätzlich als Seitenabzug abgezogen wird und worin ein Sumpfprodukt den Teilstrom d bildet.

In weiteren bevorzugten Anlagen ist der Alkylenoxidreaktor R1 in der ersten Teilanlage ein Rohrbündelreaktor und/oder der Alkylenglykolreaktor R2 in der zweiten Teilanlage ist ein Rohrreaktor.

In einer weiteren bevorzugten Anlage weist die Trennvorrichtung D mehrere hintereinander geschaltete Stufen auf, wobei in der ersten Stufe das Alkylenglykol abgetrennt wird, in der zweiten Stufe das Dialkylenglykol und in einer gegebenenfalls vorhandenen dritten Stufe das Trialkylenglykol, und wobei in der letzten Stufe Mittel für das Ausschleusen des verbliebenen Sumpfproduktes aus der Anlage vorgesehen sind; bei diesen Stufen handelt es sich ganz besonders bevorzugt um Rektifikationskolonnen.

Eine ganz besonders energieeffiziente Anlage weist eine Trennvorrichtung C auf, bei der diese Stufe aus mehreren parallel geschalteten Rektifikationskolonnen oder aus in Serie geschalteten Verdampfern besteht. Im Falle der Verwendung von Rektifikationskolonnen werden jeweils Teile des aus dem Alkylenglykolreaktor abgeführten Reaktionsgemisches eingeleitet; im Falle der Verwendung von Verdampfern wird das aus dem Alkylenglykolreaktor abgeführte Reaktionsgemisch vollständig in den ersten Verdampfer eingeleitet und anschließend in die folgenden Verdampfer. Bei dieser Variante von bevorzugten Trennvorrichtungen sind Mittel vorgesehen, um Wärmeenergie aus dem Kopf mindestens einer Rektifikationskolonne bzw. eines Verdampfers zum Erwärmen des Sumpfes mindestens einer anderen Rektifikationskolonne bzw. eines Verdampfers zu nutzen. Dem Fachmann ist die Konstruktion derartiger energieeffizienter Kolonnengruppen bzw. Verdampfer bekannt.

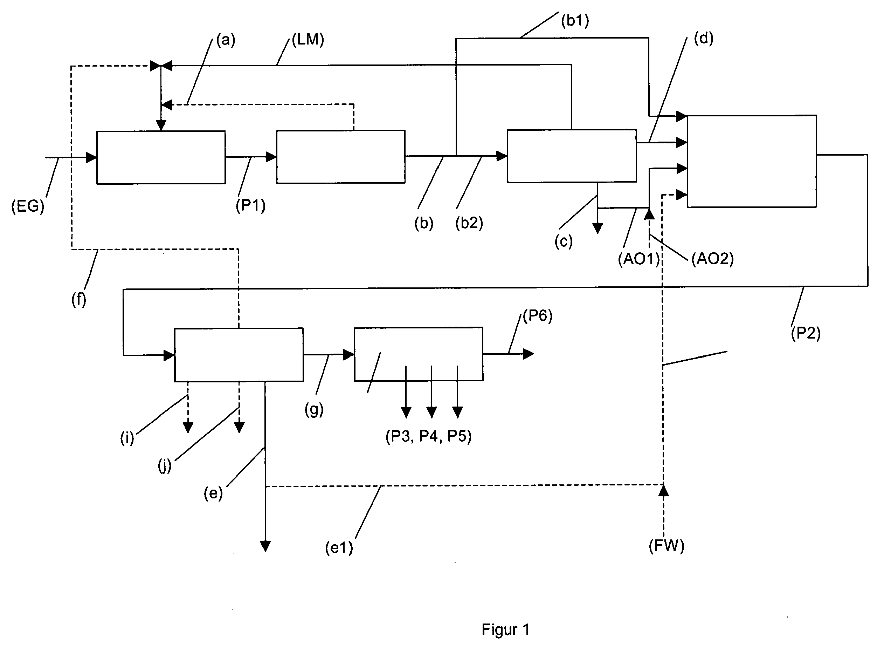

In der Figur 1 wird eine bevorzugte erfindungsgemäße Vorrichtung beispielhaft beschrieben.

Dargestellt ist eine Anlage bestehend aus einer ersten Teilanlage zur Herstellung von Alkylenoxid und aus einer zweiten Teilanlage zur Herstellung von Alkylenglykolen. Die erste Teilanlage besteht in der dargestellten Ausführungsform aus einem Alkylenoxidreaktor R1 sowie aus einer nachgeschalteten Trennvorrichtung A und einer dieser nachgeschalteten Trennvorrichtung B. Die zweite Teilanlage besteht in der dargestellten Ausführungsform aus einem Alkylenglykolreaktor R2, einer nachgeschalteten Trennvorrichtung C und daran nachgeschaltet einer Trennvorrichtung D.

In den Alkylenoxidreaktor werden Eingangsstoffe für Alkylenoxid-Reaktion (schematisch mit (EG) bezeichnet) eingeführt. Der Produktstrom P1 aus dem Alkylenoxidreaktor R1 wird in die Trennvorrichtung A eingeleitet und dort in einenTeilstrom a enthaltend im wesentlichen C₂-C₆-Olefin und gegebenenfalls weitere mit diesem abgetrennte Bestandteile des Reaktionsgemisches sowie und in einen Teilstrom b enthaltend Wasser, Alkylenoxid, Alkylenglykol und weitere Komponenten des Reaktionsgemisches sowie gegebenenfalls organisches Lösungsmittel aufgetrennt. Teilstrom a wird in den Alkylenoxidreaktor R1, gegebenenfalls nach Abtrennung der nicht-olefinischen Bestandteile, rückgeführt. Teilstrom b wird als Teilstrom b2 direkt in die Trennvorrichtung B eingeleitet und dort in einen Teilstrom c enthaltend im wesentlichen Alkylenoxid und gegebenenfalls weitere mit diesem abgetrennte Bestandteile des Reaktionsgemisches, in einen Teilstrom d enthaltend Wasser, Alkylenglykol und weitere Komponenten des Reaktionsgemisches sowie gegebenenfalls organisches Lösungsmittel und gegebenenfalls in einen Teilstrom LM enthaltend im wesentlichen organisches Lösungsmittel aufgetrennt. Teilstrom LM wird in den Alkylenoxidreaktor R1 rückgeführt. Teilstrom c kann als verkaufsfähiges Produkt aus der Anlage ausgeschleust werden oder wird vorzugsweise ganz oder teilweise als Teilstrom AO1 in den Alkylenglykolreaktor R2 eingeleitet. Alternativ kann Alkylenoxid aus anderen Quellen AO2 in den Alkylenglykolreaktor R2 eingeleitet werden. Teilstrom b kann in einer alternativen Ausführungsform als Teilstrom b1 an der Trennvorrichtung B vorbeigeleitet werden und direkt in den Alkylenglykolreaktor R2 eingeleitet werden oder Teilstrom b wird aufgeteilt in einen Teilstrom b2, der in die Trennvorrichtung B eingeleitet wird, und in einen weiteren Teilstrom b1, der direkt in den Alkylenglykolreaktor R2 eingeleitet wird.

Der Produktstrom P2 aus dem Alkylenglykolreaktor R2 wird in die Trennvorrichtung C eingeleitet und wird dort in einenTeilstrom e bestehend im wesentlichen aus dem im Reaktionsgemisch enthaltenen Wasser und gegebenenfalls weiteren mit dem Wasser abgetrennten Bestandteilen des Reaktionsgemisches, gegebenenfalls in einen Teilstrom f bestehend im wesentlichen aus dem im Reaktionsgemisch enthaltenen organischen Lösungsmittel und gegebenenfalls weiteren mit dem organischen Lösungsmittel abgetrennten Bestandteilen des Reaktionsgemisches und in einen Teilstrom g gebildet aus den Alkylenglykolen und den übrigen nicht in den Teilstrom e und nicht den Teilstrom f übergegangenen Teilen des Reaktionsgemisches sowie gegebenenfalls in einen Teilstrom i enthaltend Salze und andere im Reaktionsgemisch enthaltene Feststoffe und/oder gegebenenfalls in einen Teilstrom j enthaltend Monoalkylenglykolether aufgetrennt. Teilstrom f wird gegebenenfalls nach weiterer Aufbereitung in den Alkylenoxidreaktor R1 rückgeführt. Teilstrom e und der gegebenenfalls vorhandene Teilstrom i und/oder j werden aus der Anlage ausgeschleust. Teilstrom e wird einer nicht dargestellen Abwasseraufbereitungsanlage zugeführt. Alternativ kann Teilstrom e teilweise als Teilstrom e1 in den Alkylenglykolreaktor R2 rückgeführt werden gegebenenfalls unter Zufuhr von Frischwasser FW. Dies wird besonders dann der Fall sein, wenn der erste Anlagenteil nicht oder nur mit verringerter Produktionskapazität in Betrieb ist.

Teilstrom g wird in die Trennvorrichtung D eingeleitet und dort werden die in diesem enthaltenen Alkylenglykole und gegebenenfalls höhere Alkylenglykolether aufgetrennt. Trennvorrichtung D verlassen Teilströme verschiedener Alkylenglykole und gegebenenfalls höhere Alkylenglykolether (als P3, P4 und P5 dargestellt), beispielsweise Teilströme von Alkylenglykol, Dialkylenglykol und höhere Alkylenglykole. In der Figur sind drei Teilströme von Wertstoffen dargestellt; es können aber je nach Fahrweise der Anlage auch weniger oder mehr dieser Teilströme erzeugt werden. Außerdem wird ein Teilstrom von Schwersiedern P6 aus Trennvorrichtung D ausgeschleust.

## Patentansprüche

1. Verfahren zur Herstellung von Alkylenoxid und von Alkylenglykolen und gegebenenfalls von Alkylenglykolethern in einer integrierten Anlage mit einer ersten Teilanlage zur Herstellung von Alkylenoxid durch Umsetzung von C₂-C₆-Olefin mit einem Oxidationsmittel in flüssiger und Wasser enthaltender Phase, wobei Alkylenglykole als Nebenprodukte entstehen, und mit der ersten Teilanlage verbunden einer zweiten Teilanlage zur Herstellung von Alkylenglykolen und gegebenenfalls von Alkylenglykolether durch Umsetzung von Alkylenoxid mit Wasser und gegebenenfalls mit Alkohol in flüssiger Phase, wobei das aus der ersten Teilanlage stammende und zumindest Wasser und Alkylenglykole sowie gegebenenfalls Alkylenglykolether aufweisende Reaktionsgemisch, aus der ersten Teilanlage in die zweite Teilanlage eingeleitet wird, die erste Teilanlage mindestens einen Alkylenoxidreaktor R1 sowie eine nachgeschaltete Trennvorrichtung A und gegebenenfalls eine daran nachge-schaltete Trennvorrichtung B, welche hauptsächlich aus Rektifikationskolonnen bestehen, aufweist und mit der ersten Teilanlage verbunden eine zweite Teilanlage zur Herstellung von Alkylenglykolen, die mindestens einen Alkylenglykolreaktor R2 sowie mindestens eine nachgeschaltete Trennvorrichtung C und daran nachgeschaltet mindestens eine Trennvorrichtung D aufweist, wobei das Verfahren folgende Schritte aufweist:
i) Erzeugen von Alkylenoxid durch Umsetzung von C₂-C₆-Olefin mit einem Oxidationsmittel in flüssiger Phase in der ersten Teilanlage,
ii) Auftrennen des den Alkylenoxidreaktor R1 verlassenden Reaktionsgemisches in der Trennvorrichtung A in einen Teilstrom a enthaltend C₂-C₆-Olefin und gegebenenfalls weitere mit diesem abgetrennte Bestandteile des Reaktionsgemisches und in einen Teilstrom b enthaltend Wasser, Alkylenoxid, Alkylenglykol und weitere Komponenten des Reaktionsgemisches sowie gegebenenfalls organisches Lösungsmittel,
iii) gegebenenfalls Auftrennen des Teilstromes b in der Trennvorrichtung B in einen Teilstrom c enthaltend Alkylenoxid und gegebenenfalls weitere mit diesem abgetrennte Bestandteile des Reaktionsgemisches und in einen Teilstrom d enthaltend Wasser, Alkylenglykol und weitere Komponenten des Reaktionsgemisches sowie gegebenenfalls organisches Lösungsmittel,
iv) Erzeugen von Alkylenglykolen durch Umsetzung von Alkylenoxid mit Wasser in wässriger Phase in der zweiten Teilanlage, indem
v) der Teilstrom b aus der Trennvorrichtung A und/oder der Teilstrom d aus der Trennvorrichtung B, gegebenenfalls nach einer Einstellung des pH-Werts, in den Alkylenglykolreaktor R2 geleitet wird,
vi) Alkylenoxid aus Teilstrom c und/oder aus anderern Quellen in den Alkylenglykolreaktor R2 geleitet wird, wobei dieser Schritt im Falle des Einleitens des Teilstromes b aus der Trennvorrichtung A in den Alkylenglykolreaktor R2 entfallen kann,
vii) das den Alkylenglykolreaktor R2 verlassende und Alkylenglykole und Alkylenglykolether enthaltende Reaktionsgemisch in der Trennvorrichtung C in einen Teilstrom e bestehend aus dem im Reaktionsgemisch enthaltenen Wasser und gegebenenfalls weiteren mit dem Wasser abgetrennten Bestandteilen des Reaktionsgemisches sowie gegebenenfalls zusätzlich in einen Teilstrom f bestehend aus dem im Reaktionsgemisch enthaltenen organischen Lösungsmittel und gegebenenfalls weiteren mit dem organischen Lösungsmittel abgetrennten Bestandteilen des Reaktionsgemisches und in einen Teilstrom g gebildet aus den Alkylenglykolen und den übrigen nicht in den Teilstrom e und gegebenenfalls nicht in den Teilstrom f übergegangenen Teilen des Reaktionsgemisches sowie gegebenenfalls in einen Teilstrom i enthaltend Salze und andere Feststoffe aus dem Reaktionsgemisch und gegebenenfalls in einen Teilstrom j enthaltend Monoalkylenglykyolether aufgetrennt wird,
viii) zumindest ein Teil des Teilstromes e aus der Anlage ausgeschleust wird und vorzugsweise in eine Abwasserreinigungsanlage verbracht wird und der gegebenenfalls verbliebene Teil des Teilstromes e in den Eingang des Alkylenglykolreaktors R2 rückgeführt wird,
ix) der gegebenenfalls vorliegende Teilstrom f gegebenenfalls nach weiterer Aufarbeitung in den Alkylenoxidreaktor R1 zurückgeführt wird, und
x) die im Teilstrom g enthaltenen Alkylenglykole in der Trennvorrichtung D aufgetrennt werden.

2. Verfahren nach Anspruch 1, **dadurch gekennzeichnet, dass** in der ersten Teilanlage Propylen mit einem Peroxid oder Hydroperoxid in wässriger oder in wässrigalkoholischer Phase katalytisch zu Propylenoxid umgesetzt werden, wobei vorzugsweise als Peroxid Wasserstoffperoxid verwendet wird.

3. Verfahren nach einem der Ansprüche 1 bis 2, **dadurch gekennzeichnet, dass** als Trennvorrichtung A mindestens ein Flash oder Verdampfer verwendet wird, und dass als Trennvorrichtung B mindestens eine Rektifikationskolonne verwendet wird, wobei zumindest ein Teil des Alkylenoxids über Kopf abgezogen wird, gegebenenfalls ein Teil des Alkylenoxids als Seitenabzug und dass ein Sumpfprodukt den Teilstrom d bildet.

4. Verfahren nach Anspruch 1, **dadurch gekennzeichnet, dass** das Erzeugen von Alkylenglykolen durch katalytische Umsetzung von Alkylenoxid mit Wasser in wässriger Phase im Alkylenglykolreaktor erfolgt, insbesondere durch katalytische Umsetzung von Alkylenoxid mit Wasser in saurer wässriger Phase im Alkylenglykolreaktor, wobei als Katalysator eine Säure oder ein saurer Ionenaustauscher verwendet wird.

5. Verfahren nach Anspruch 1, **dadurch gekennzeichnet, dass** der Teilstrom b in der Trennvorrichtung B in einen Teilstrom c und in einen Teilstrom d aufgetrennt wird, dass der Teilstrom d und zumindest ein Teil des Teilstromes c aus der Trennvorrichtung B in den Alkylenglykolreaktor eingeleitet werden, wobei gegebenenfalls der Teilstrom d aus der Trennvorrichtung B vor dem Einleiten in den Alkylenglykolreaktor R2 mit einem Teil des Teilstromes e aus der Trennvorrichtung C kombiniert wird.

6. Verfahren nach Anspruch 1, **dadurch gekennzeichnet, dass** die Auftrennung des den Alkylenglykolreaktor R2 verlassenden und Alkylenglykole enthaltenden Reaktionsgemisches in der Trennvorrichtung C so erfolgt, dass der Teilstrom e aus dem im Reaktionsgemisch enthaltenen Wasser besteht und gegebenenfalls aus Ethern, die sich aus Teilen des Alkylenoxids und anderen Bestandteilen des Reaktionsgemisches gebildet haben.

7. Verfahren nach Anspruch 6, **dadurch gekennzeichnet, dass** die Trennvorrichtung C aus mehreren parallel geschalteten Rektifikationskolonnen sowie vorzugsweise aus mindestens einer nachgeschalteten Rektifikationskolonne besteht, wobei in die parallel geschalteten Rektifikationskolonnen jeweils Teile des aus dem Alkylenglykolreaktor R2 ausgeschleusten Reaktionsgemisches eingeleitet werden, wobei Wärmeenergie aus dem Kopf mindestens einer Rektifikationskolonne zum Erwärmen des Sumpfes mindestens einer anderen Rektifikationskolonne genutzt wird und die Rektifikationskolonnen bei unterschiedlichen Drucken betrieben werden, so dass das Kopfprodukt jeweils aus Wasser und gegenbenfalls aus Ethern und Resten von organischem Lösungsmittel und aus Resten von Alkylenglykol besteht, und wobei die vereinigten Kopfprodukte vorzugsweise aus den parallel geschalteten Rektifikationskolonnen in die mindestens eine nachgeschaltete Rektifikationskolonne eingeleitet werden und dort das darin enthaltene Wasser abgetrennt wird.

8. Verfahren nach Anspruch 6, **dadurch gekennzeichnet, dass** die Trennvorrichtung C aus mehreren in Serie geschalteten Verdampfern besteht, wobei die Kondensationswärme des aus mindestens einem Verdampfer austretenden Dampfstromes zum Erwärmen des jeweils nächsten Verdampfers genutzt wird und die Verdampfer bei unterschiedlichen Drucken betrieben werden, so dass das Kopfprodukt jeweils aus Wasser und gegebenenfalls aus Ethern und Resten von organischem Lösungsmittel und aus Resten von Alkylenglykol besteht.

9. Verfahren nach Anspruch 1, **dadurch gekennzeichnet, dass** eine Trennvorrichtung D für Alkylenglykol mit mehreren hintereinander geschalteten Stufen verwendet wird, wobei in der ersten Stufe das Alkylenglykol abgetrennt wird, in der zweiten Stufe das Dialkylenglykol und in einer gegebenenfalls vorhandenen dritten Stufe das Trialkylenglykol gegebenenfalls zusammen mit weiteren Mittel- oder Schwersiedern, und dass das verbliebene Sumpfprodukt aus der Anlage ausgeschleust wird, wobei die Stufen vorzugsweise aus Rektifikationskolonnen gebildet werden.

10. Anlage zur Herstellung von Alkylenoxid und von Alkylenglykolen und gegebenenfalls von Alkylenglykolethern mit folgenden Elementen:
A) erste Teilanlage zur Herstellung von Alkylenoxid ,
B) zweite Teilanlage zur Herstellung von Alkylenglykolen und gegebenenfalls von Alkylenglykolethern, wobei
die erste Teilanlage mit der zweiten Teilanlage verbunden ist und mindestens eine Leitung aufweist, durch welche das aus der ersten Teilanlage stammende und zumindest Wasser, Alkylenglykole und Alkylenglykolether aufweisende Reaktionsgemisch aus der ersten Teilanlage in die zweite Teilanlage eingeleitet wird, sowie den Elementen C) bis F), wobei
C) die erste Teilanlage mindestens einen Alkylenoxidreaktor R1 sowie eine nachgeschaltete Trennvorrichtung A zur Auftrennung des den Alkylenoxidreaktor R1 verlassenden Reaktionsgemisches in einen Teilstrom a enthaltend im wesentlichen C₂-C₆-Olefin und gegebenenfalls weitere mit diesem abgetrennte Bestandteile des Reaktionsgemisches und in einen Teilstrom b enthaltend Wasser, Alkylenoxid, Alkylenglykol und weitere Komponenten des Reaktionsgemisches sowie gegebenenfalls organisches Lösungsmittel, und gegebenenfalls eine der Trennvorrichtung A nachgeschaltete Trennvorrichtung B zum Auftrennen des Teilstromes b in einen Teilstrom c enthaltend Alkylenoxid und gegebenenfalls weitere mit diesem abgetrennte Bestandteile des Reaktionsgemisches und in einen Teilstrom d enthaltend Wasser, Alkylenglykol und weitere Komponenten des Reaktionsgemisches sowie gegebenenfalls organisches Lösungsmittel,
D) die zweite Teilanlage mindestens einen Alkylenglykolreaktor R2 sowie mindestens eine nachgeschaltete Trennvorrichtung C und daran nachgeschaltet mindestens eine Trennvorrichtung D aufweist, wobei in der Trennvorrichtung C das den Alkylenglykolreaktor R2 verlassende Reaktionsgemisch in einen Teilstrom e bestehend aus dem im Reaktionsgemisch enthaltenen Wasser und gegebenenfalls weiteren mit dem Wasser abgetrennten Bestandteilen des Reaktionsgemisches, sowie gegebenenfalls zusätzlich in einen Teilstrom f bestehend aus dem im Reaktionsgemisch enthaltenen organischen Lösungsmittel und gegebenenfalls weiteren mit dem organischen Lösungsmittel abgetrennten Bestandteilen des Reaktionsgemisches und in einen Teilstrom g gebildet aus den Alkylenglykolen und den übrigen nicht in den Teilstrom e und nicht den Teilstrom f übergegangenen Teilen des Reaktionsgemisches sowie gegebenenfalls in einen Teilstorm i enthaltend Salze und andere Feststoffe aus dem Reaktionsgemisch und gegebenenfalls in einen Teilstrom j enthaltend Monoalkylenglykolether aufgetrennt wird,
E) der Alkylenglykolreaktor mindestens eine Leitung E) für die Zuführung von Alkylenoxid aufweist, wobei diese Leitung E) im Falle der Zuführung des Teilstromes b in den Alkylenglykolreaktor entfallen kann, und wobei
F) mindestens eine Leitung F) vorgesehen ist, durch welche zumindest ein Teil des Teilstroms b und/oder des Teilstroms d aus der ersten Teilanlage, gegebenenfalls nach Einstellung des pH-Wertes, in den Alkylenglykolreaktor R2 der zweiten Teilanlage eingeleitet wird.

11. Anlage nach Anspruch 10, **dadurch gekennzeichnet, dass** eine Leitung G) vorgesehen ist, durch die zumindest ein Teil des Teilstromes e in eine Abwasserreinigungsanlage verbracht wird und/oder dass eine Leitung H) vorgesehen ist, durch die der Teilstrom f, vorzugsweise nach weiterer Aufreinigung, in den Alkylenoxidreaktor zurückgeführt wird und/oder dass eine Leitung l) vorgesehen ist, durch die zumindest ein Teil des Teilstromes a in den Alkylenoxidreaktor rückgeführt wird und/oder dass eine Leitung J) vorgesehen ist, durch die ein Teil des Teilstromes e in den Eingang des Alkylenglykolreaktors rückgeführt wird und/oder dass eine Leitung K) vorgesehen ist, durch welche Salze und andere im Reaktionsgemisch enthaltene Feststoffe als Teilstrom i aus der Anlage ausgeschleust werden und/oder dass eine Leitung L) vorgesehen ist, durch welche Monoalkylenglykolether als Teilstrom j aus der Anlage ausgeschleust werden.

12. Anlage nach Anspruch 11, **dadurch gekennzeichnet, dass** Leitungen F) und J) vorgesehen sind und dass Mittel vorgesehen sind, mit der die Menge der über Leitungen F) und J) in den Alkylenglykolreaktor eingeführten Teilströme geregelt werden kann.

13. Anlage nach Anspruch 10, **dadurch gekennzeichnet, dass** die Trennvorrichtung A und die Trennvorrichtung B vorhanden sind und jeweils eine Rektifikationskolonne oder eine Gruppe von miteinander verschalteten Rektifikationskolonnen sind, wobei vorzugsweise in der Trennvorrichtung B das Alkylenoxid über den Kolonnenkopf sowie gegebenenfalls zusätzlich als Seitenabzug abgezogen wird und ein Sumpfprodukt den Teilstrom d bildet.

14. Anlage nach Anspruch 10, **dadurch gekennzeichnet, dass** der Alkylenoxidreaktor R1 ein Rohrbündelreaktor ist und/oder dass der Alkylenglykolreaktor R2 ein Rohrreaktor ist.

15. Anlage nach Anspruch 10, **dadurch gekennzeichnet, dass** die Trennvorrichtung C mehrere parallel geschaltete Rektifikationskolonnen aufweist, in die jeweils Teile des aus dem Alkylenglykolreaktor austretenden Reaktionsgemisches eingeführt werden, und dass Mittel vorgesehen sind, um Wäremenergie aus dem Kopf mindestens einer Rektifikationskolonne zum Erwärmen des Sumpfes mindestens einer anderen Rektifikationskolonne zu nutzen, oder dass die Trennvorrichtung C mehrere in Serie geschaltete Verdampfer aufweist, dass das aus dem Alkylenglykolreaktor austretende Reaktionsgemisch in den ersten Verdampfer eingeführt wird, und dass Mittel vorgesehen sind, um die Kondensationswärme des aus mindestens einem Verdampfer austretenden Dampfstromes zum Erwärmen eines anderen, vorzugsweise des jeweils nächsten Verdampfers zu nutzen.

16. Anlage nach Anspruch 10, **dadurch gekennzeichnet, dass** die Trennvorrichtung C mindestens eine Rektifikationskolonne aufweist, in der 90 bis 98 % des vorhandenen Wassers abgetrennt werden und dass eine weitere Rektifikationskolonne vorhanden ist, in welcher der Rest des Wassers abgetrennt wird.

17. Anlage nach Anspruch 10, **dadurch gekennzeichnet, dass** die Trennvorrichtung D mehrere hintereinander geschaltete Stufen aufweist, wobei in der ersten Stufe das Alkylenglykol abgetrennt wird, in der zweiten Stufe das Dialkylenglykol und in einer gegebenenfalls vorhandenen dritten Stufe das Trialkylenglykol und gegebenenfalls weitere Mittel- oder Schwersieder, und wobei in der letzten Stufe Mittel für das Ausschleusen des verbliebenen Sumpfproduktes aus der Anlage vorgesehen sind, wobei die Stufen der Trennvorrichtung D vorzugsweise Rektifikationskolonnen sind.

## Claims

1. Process for preparing alkylene oxide and alkylene glycols and, if appropriate, alkylene glycol ethers in an integrated plant having a first subplant for preparing alkylene oxide by reacting C₂-C₆-olefin with an oxidant in a liquid and water-containing phase, with alkylene glycols being formed as by-products, and a second subplant for preparing alkylene glycols and, if appropriate, alkylene glycol ethers by reacting alkylene oxide with water and, if appropriate, with alcohol in the liquid phase being connected to the first subplant, wherein the reaction mixture which originates from the first subplant and comprises at least water and alkylene glycols and, if appropriate, alkylene glycol ethers is introduced from the first subplant into the second subplant, the first subplant has at least one alkylene oxide reactor R1 and a downstream separation apparatus A and, if appropriate, a separation apparatus B downstream of A, which mainly comprises rectification columns, and, connected to the first subplant, a second subplant for preparing alkylene glycols, which has at least one alkylene glycol reactor R2 and at least one downstream separation apparatus C and at least one separation apparatus D located downstream of the third, wherein the process comprises the following steps:
i) production of alkylene oxide by reaction of C₂-C₆-olefin with an oxidant in the liquid phase in the first subplant,
ii) separation of the reaction mixture leaving the alkylene oxide reactor R1 in the separation apparatus A into a substream a comprising C₂-C₆-olefin and, if appropriate, further constituents of the reaction mixture which have been separated off with this and a substream b comprising water, alkylene oxide, alkylene glycol and further components of the reaction mixture and also, if appropriate, organic solvent,
iii) if appropriate, separation of the substream b in the separation apparatus B into a substream c comprising alkylene oxide and, if appropriate, further constituents of the reaction mixture which have been separated off with this and a substream d comprising water, alkylene glycol and further components of the reaction mixture and also, if appropriate, organic solvent,
iv) production of alkylene glycols by reaction of alkylene oxide with water in the aqueous phase in the second subplant, by
v) introducing the substream b from the separation apparatus A or the substream d from the separation apparatus B, if appropriate after adjusting the pH, into the alkylene glycol reactor R2,
vi) introducing alkylene oxide from substream c and/or from other sources into the alkylene glycol reactor R2, with this step being able to be omitted if the substream b from the separation apparatus A is introduced into the alkylene glycol reactor R2,
vii) separating the alkylene glycol- and alkylene glycol ether-containing reaction mixture leaving the alkylene glycol reactor R2 in the separation apparatus C into a substream e consisting of the water present in the reaction mixture and, if appropriate, further constituents of the reaction mixture which have been separated off with the water and also, if appropriate, additionally a substream f consisting of the organic solvent present in the reaction mixture and, if appropriate, further constituents of the reaction mixture which have been separated off with the organic solvent and a substream g formed by the alkylene glycols and the other components of the reaction mixture which have not gone over into the substream e and, if appropriate, not into the substream f and also, if appropriate, into a substream i containing salts and other solids from the reaction mixture and, if appropriate, into a substream j containing monoalkylene glycol ethers,
viii) discharging at least part of the substream e from the plant and preferably introducing it into a wastewater purification plant and recirculating any remaining part of the substream e to the inlet of the alkylene glycol reactor R2,
ix) recirculating any substream f present, if appropriate after further processing to the alkylene oxide reactor R1 and
x) separating the alkylene glycols present in the substream g in the separation apparatus D.

2. Process according to Claim 1, **characterized in that**, in the first subplant, propylene is catalytically reacted with a peroxide or hydroperoxide in an aqueous or aqueous-alcoholic phase to form propylene oxide, with hydrogen peroxide preferably being used as peroxide.

3. Process according to either of Claims 1 and 2, **characterized in that** at least one flash vessel or vaporizer is used as separation apparatus A and **in that** at least one rectification column is used as separation apparatus B, with at least part of the alkylene oxide being taken off at the top, if appropriate part of the alkylene oxide being taken off as side offtake stream and **in that** a bottom product forms the substream d.

4. Process according to Claim 1, **characterized in that** the production of alkylene glycols is carried out by catalytic reaction of alkylene oxide with water in an aqueous phase in the alkylene glycol reactor, in particular by catalytic reaction of alkylene oxide with water in an acidic aqueous phase in the alkylene glycol reactor, with an acid or an acidic ion exchanger being used as catalyst.

5. Process according to Claim 1, **characterized in that** the substream b is separated in the separation apparatus B into a substream c and a substream d, **in that** the substream d and at least part of the substream c are conveyed from the separation apparatus B into the alkylene glycol reactor, in which, if appropriate, the substream d from the separation apparatus B is combined with part of the substream e from the separation apparatus C before being introduced into the alkylene glycol reactor R2.

6. Process according to Claim 1, **characterized in that** the fractionation of the reaction mixture containing alkylene glycols leaving the alkylene glycol reactor R2 in the separation apparatus C is carried out in such a way that the substream e consists of the water present in the reaction mixture and possibly ethers which have been formed from parts of the alkylene oxide and other constituents of the reaction mixture.

7. Process according to Claim 6, **characterized in that** the separation apparatus C comprises a plurality of rectification columns which are connected in parallel and also preferably comprises at least one downstream rectification column, where, in the rectification columns connected in parallel, in each case parts of the reaction mixture discharged from the alkylene glycol reactor R2 are introduced, with heat energy from the top of at least one rectification column being utilized for heating the bottom of at least one other rectification column and the rectification columns being operated at different pressures so that the overhead product in each case comprises water and possibly ethers and residues of organic solvent and residues of alkylene glycol and the combined overhead products from the rectification columns connected in parallel preferably being introduced into the at least one downstream rectification column and the water present therein being separated off there.

8. Process according to Claim 6, **characterized in that** the separation apparatus C comprises a plurality of vaporizers connected in series, with the heat of condensation of the vapor stream leaving at least one vaporizer being utilized for heating the next vaporizer and the vaporizers being operated at different pressures so that the overhead product in each case comprises water and possibly ethers and residues of organic solvent and residues of alkylene glycol.

9. Process according to Claim 1, **characterized in that** a separation apparatus D for alkylene glycol having a plurality of stages connected in series is used, with the alkylene glycol being separated off in the first stage, the dialkylene glycol being separated off in the second stage and the trialkylene glycol being separated off in an optionally present third stage, if appropriate, along with further intermediate or high boilers, and **in that** the remaining bottom product is discharged from the plant, the stages preferably being formed from rectification columns.

10. Plant for preparing alkylene oxide and alkylene glycols and, if appropriate, alkylene glycol ethers, which comprises the following elements:
A) a first subplant for preparing alkylene oxide,
B) a second subplant for preparing alkylene glycols and, if appropriate, alkylene glycol ethers, where
the first subplant is connected to the second subplant and has at least one line through which the reaction mixture which originates from the first subplant and comprises at least water, alkylene glycols and alkylene glycol ethers is introduced into the second subplant, and also the elements C) to F), where
C) the first subplant comprises at least one alkylene glycol reactor R1 and also a downstream separation apparatus A for separating the reaction mixture leaving the alkylene oxide reactor R1 into a substream a comprising essentially C₂-C₆-olefin and possibly further constituents of the reaction mixture which have been separated off with the olefin and a substream b containing water, alkylene oxide, alkylene glycol and further components of the reaction mixture and also, if appropriate, organic solvent and, if appropriate, a separation apparatus B downstream of the separation apparatus A for separating the substream b into a substream c comprising alkylene oxide and possibly further constituents of the reaction mixture which have been separated off with the alkylene oxide and a substream d containing water, alkylene glycol and further components of the reaction mixture and also, if appropriate, organic solvent,
D) the second subplant comprises at least one alkylene glycol reactor R2 and also at least one downstream separation apparatus C and, downstream thereof, at least one separation apparatus D, where the reaction mixture leaving the alkylene glycol reactor R2 is separated in the separation apparatus C into a substream e consisting of the water present in the reaction mixture and possibly further constituents of the reaction mixture which have been separated off with the water and, if appropriate, additionally a substream f consisting of the organic solvent present in the reaction mixture and possibly further constituents of the reaction mixture which have been separated off with the organic solvent and a substream g formed by the alkylene glycols and the other parts of the reaction mixture which have not gone over into the substream e and not gone over into the substream f and, if appropriate, into a substream i containing salts and other solids from the reaction mixture and, if appropriate, into a substream j containing monoalkylene glycol ether,
E) the alkylene glycol reactor has at least one line E) for the introduction of alkylene oxide, where this line E) can be omitted if the substream b is fed into the alkylene glycol reactor, and
F) at least one line F) through which at least part of the substream b and/or the substream d from the first subplant, if appropriate, after adjusting the pH, is introduced into the alkylene glycol reactor R2 of the second subplant is provided.

11. Plant according to Claim 10, **characterized in that** a line G) through which at least part of the substream e is brought to a wastewater purification plant is provided and/or **in that** a line H) through which the substream f is, preferably after further purification, recirculated to the alkylene oxide reactor is provided and/or **in that** a line I) through which at least part of the substream a is recirculated to the alkylene oxide reactor is provided and/or **in that** a line J) through which part of the substream e is recirculated to the inlet of the alkylene glycol reactor is provided and/or **in that** a line K) through which salts and other solids present in the reaction mixture are discharged from the plant as substream i is provided and/or **in that** a line L) through which monoalkylene glycol ethers are discharged from the plant as substream j is provided.

12. Plant according to Claim 11, **characterized in that** lines F) and J) are provided and **in that** means which allow the amount of the substreams introduced via lines F) and J) into the alkylene glycol reactor to be regulated are provided.

13. Plant according to Claim 10, **characterized in that** the separation apparatus A and the separation apparatus B are present and are each a rectification column or a group of rectification columns connected to one another, where, preferably in the separation apparatus B, the alkylene oxide is taken off at the top of the column and, if appropriate, additionally as side offtake stream and a bottom product forms the substream d.

14. Plant according to Claim 10, **characterized in that** the alkylene oxide reactor R1 is a shell-and-tube reactor and/or **in that** the alkylene glycol reactor R2 is a tube reactor.

15. Plant according to Claim 10, **characterized in that** the separation apparatus C has a plurality of rectification columns which are connected in parallel and into each of which parts of the reaction mixture leaving the alkylene glycol reactor are introduced and **in that** means of utilizing heat energy from the top of at least one rectification column for heating the bottom of at least one other rectification column are provided, or **in that** the separation apparatus C has a plurality of vaporizers connected in series, **in that** the reaction mixture leaving the alkylene glycol reactor is introduced into the first vaporizer and **in that** means of utilizing the heat of condensation of the vapor stream leaving at least one vaporizer for heating another, preferably the next vaporizer are provided.

16. Plant according to Claim 10, **characterized in that** the separation apparatus C has at least one rectification column in which from 90 to 98% of the water present is separated off and **in that** there is a further rectification column in which the remainder of the water is separated off.

17. Plant according to Claim 10, **characterized in that** the separation apparatus D has a plurality of stages connected in series, with the alkylene glycol being separated off in the first stage, the dialkylene glycol being separated off in the second stage and the trialkylene glycol being separated off in an optionally present third stage and, if appropriate, further intermediate or high boilers, and means of discharging the remaining bottom product from the plant being provided in the last stage, where the stages of the separation apparatus D are preferably rectification columns.

## Revendications

1. Procédé de fabrication d'oxyde d'alkylène et d'alkylène glycols et éventuellement d'éthers d'alkylène glycols dans une unité intégrée comprenant une première unité partielle pour la fabrication d'oxyde d'alkylène par mise en réaction d'une oléfine en C₂-C₆ avec un oxydant dans une phase liquide et contenant de l'eau, des alkylène glycols se formant en tant que produits secondaires, et la première unité partielle étant raccordée à une seconde unité partielle pour la fabrication d'alkylène glycols et éventuellement d'éthers d'alkylène glycols par mise en réaction d'oxyde d'alkylène avec de l'eau et éventuellement avec un alcool en phase liquide, le mélange réactionnel issu de la première unité partielle et comprenant au moins de l'eau et des alkylène glycols, ainsi qu'éventuellement des éthers d'alkylène glycols, étant introduit depuis la première unité partielle dans la seconde unité partielle, la première unité partielle comprenant au moins un réacteur d'oxyde d'alkylène R1, ainsi qu'un dispositif de séparation A raccordé en aval et éventuellement un dispositif de séparation B raccordé en aval de celui-ci, qui sont principalement constitués de colonnes de rectification, et la seconde unité partielle raccordée à la première unité partielle, pour la fabrication d'alkylène glycols, comprenant au moins un réacteur d'alkylène glycol R2, ainsi qu'au moins un dispositif de séparation C raccordé en aval et au moins un dispositif de séparation D raccordé en aval à celui-ci, le procédé comprenant les étapes suivantes :
i) la formation d'oxyde d'alkylène par mise en réaction d'une oléfine en C₂-C₆ avec un oxydant en phase liquide dans la première unité partielle,
ii) la séparation du mélange réactionnel quittant le réacteur d'oxyde d'alkylène R1 dans le dispositif de séparation A en un courant partiel a contenant une oléfine en C₂-C₆ et éventuellement d'autres constituants du mélange réactionnel séparés avec celle-ci et en un courant partiel b contenant de l'eau, de l'oxyde d'alkylène, de l'alkylène glycol et d'autres composants du mélange réactionnel, ainsi qu'éventuellement un solvant organique,
iii) éventuellement la séparation du courant partiel b dans le dispositif de séparation B en un courant partiel c contenant de l'oxyde d'alkylène et éventuellement d'autres constituants du mélange réactionnel séparés avec celui-ci et en un courant partiel d contenant de l'eau, de l'alkylène glycol et d'autres composants du mélange réactionnel, ainsi qu'éventuellement un solvant organique,
iv) la formation d'alkylène glycols par mise en réaction d'oxyde d'alkylène avec de l'eau en phase aqueuse dans la seconde unité partielle, selon laquelle
v) le courant partiel b issu du dispositif de séparation A et/ou le courant partiel d issu du dispositif de séparation B, éventuellement après ajustement du pH, sont introduits dans le réacteur d'alkylène glycol R2,
vi) l'oxyde d'alkylène issu du courant partiel c et/ou d'autres sources est introduit dans le réacteur d'alkylène glycol R2, cette étape pouvant être omise en cas d'introduction du courant partiel b issu du dispositif de séparation A dans le réacteur d'alkylène glycol R2,
vii) le mélange réactionnel quittant le réacteur d'alkylène glycol R2 et contenant des alkylène glycols et des éthers d'alkylène glycols est séparé dans le dispositif de séparation C en un courant partiel e constitué par l'eau contenue dans le mélange réactionnel et éventuellement d'autres constituants du mélange réactionnel séparés avec l'eau, ainsi qu'éventuellement également en un courant partiel f constitué par le solvant organique contenu dans le mélange réactionnel et éventuellement d'autres constituants du mélange réactionnel séparés avec le solvant organique, et en un courant partiel g formé par les alkylène glycols et les autres parties du mélange réactionnel qui ne sont pas passées dans le courant partiel e et éventuellement pas dans le courant partiel f, ainsi qu'éventuellement en un courant partiel i contenant des sels et d'autres solides issus du mélange réactionnel, et éventuellement en un courant partiel j contenant des éthers de monoalkylène glycol,
viii) au moins une partie du courant partiel e est déchargée de l'unité et de préférence transférée dans une unité de purification de l'eau résiduaire, et la partie éventuellement restante du courant partiel e est recyclée dans l'entrée du réacteur d'alkylène glycol R2,
ix) le courant partiel f éventuellement présent, éventuellement après un traitement supplémentaire, est recyclé dans le réacteur d'oxyde d'alkylène R1, et
x) les alkylène glycols contenus dans le courant partiel g sont séparés dans le dispositif de séparation D.

2. Procédé selon la revendication 1, **caractérisé en ce que**, dans la première unité partielle, du propylène est mis en réaction avec un peroxyde ou hydroperoxyde dans une phase aqueuse ou aqueuse-alcoolique, sous catalyse, pour former de l'oxyde de propylène, du peroxyde d'hydrogène étant utilisé de préférence en tant que peroxyde.

3. Procédé selon l'une quelconque des revendications 1 à 2, **caractérisé en ce qu'**au moins un dispositif de détente ou un évaporateur est utilisé en tant que dispositif de séparation A, et **en ce qu'**au moins une colonne de rectification est utilisée en tant que dispositif de séparation B, au moins une partie de l'oxyde d'alkylène étant soutiré par la tête, éventuellement une partie de l'oxyde d'alkylène en tant que soutirage latéral, et **en ce qu'**un produit de fond forme le courant partiel d.

4. Procédé selon la revendication 1, **caractérisé en ce que** la formation d'alkylène glycols a lieu par mise en réaction catalytique d'oxyde d'alkylène avec de l'eau en phase aqueuse dans le réacteur d'alkylène glycol, notamment par mise en réaction catalytique d'un oxyde d'alkylène avec de l'eau en phase aqueuse acide dans le réacteur d'alkylène glycol, un acide ou un échangeur d'ions acide étant utilisé en tant que catalyseur.

5. Procédé selon la revendication 1, **caractérisé en ce que** le courant partiel b est séparé dans le dispositif de séparation B en un courant partiel c et en un courant partiel d, **en ce que** le courant partiel d et au moins une partie du courant partiel c sont introduits depuis le dispositif de séparation B dans le réacteur d'alkylène glycol, le courant partiel d issu du dispositif de séparation B étant éventuellement combiné avec une partie du courant partiel e issu du dispositif de séparation C avant l'introduction dans le réacteur d'alkylène glycol R2.

6. Procédé selon la revendication 1, **caractérisé en ce que** la séparation du mélange réactionnel quittant le réacteur d'alkylène glycol R2 et contenant des alkylène glycols dans le dispositif de séparation C a lieu de sorte que le courant partiel e soit constitué par l'eau contenue dans le mélange réactionnel et éventuellement par des éthers, qui se sont formés à partir de parties de l'oxyde d'alkylène et d'autres constituants du mélange réactionnel.

7. Procédé selon la revendication 6, **caractérisé en ce que** le dispositif de séparation C est constitué de plusieurs colonnes de rectification raccordées en parallèle et de préférence d'au moins une colonne de rectification raccordée en aval, des parties du mélange réactionnel déchargé du réacteur d'alkylène glycol R2 étant introduites dans chacune des colonnes de rectification raccordées en parallèle, l'énergie thermique issue de la tête d'au moins une colonne de rectification étant utilisée pour le chauffage du fond d'au moins une autre colonne de rectification et les colonnes de rectification étant exploitées à des pressions différentes, de sorte que le produit de tête soit constitué à chaque fois d'eau et éventuellement d'éthers et de résidus de solvant organique et de résidus d'alkylène glycol, et les produits de tête réunis étant de préférence introduits depuis les colonnes de rectification raccordées en parallèle dans ladite au moins une colonne de rectification raccordée en aval et l'eau qu'ils contiennent y étant séparée.

8. Procédé selon la revendication 6, **caractérisé en ce que** le dispositif de séparation C est constitué de plusieurs évaporateurs raccordés en série, la chaleur de condensation du courant de vapeur sortant d'au moins un évaporateur étant utilisée pour le chauffage de l'évaporateur suivant, et les évaporateurs étant exploités à des pressions différentes, de sorte que le produit de tête soit à chaque fois constitué d'eau et éventuellement d'éthers et de résidus de solvant organique et de résidus d'alkylène glycol.

9. Procédé selon la revendication 1, **caractérisé en ce qu'**un dispositif de séparation D pour l'alkylène glycol comprenant plusieurs étapes raccordées les unes après les autres est utilisé, l'alkylène glycol étant séparé dans la première étape, le dialkylène glycol dans la deuxième étape et le trialkylène glycol dans une troisième étape éventuellement présente, éventuellement conjointement avec d'autres composants de point d'ébullition moyen ou élevé, et **en ce que** le produit de fond restant est déchargé de l'unité, les étapes étant de préférence formées par des colonnes de rectification.

10. Unité pour la fabrication d'oxyde d'alkylène et d'alkylène glycols et éventuellement d'éthers d'alkylène glycols, comprenant les éléments suivantes :
A) une première unité partielle pour la fabrication d'oxyde d'alkylène,
B) une seconde unité partielle pour la fabrication d'alkylène glycols et éventuellement d'éthers d'alkylène glycols,
la première unité partielle étant raccordée avec la seconde unité partielle et comprenant au moins une conduite, qui introduit le mélange réactionnel issu de la première unité partielle et comprenant au moins de l'eau, des alkylène glycols et des éthers d'alkylène glycols, à partir de la première unité partielle dans la seconde unité partielle, ainsi que les éléments C) à F),
C) la première unité partielle comprenant au moins un réacteur d'oxyde d'alkylène R1, ainsi qu'un dispositif de séparation A raccordé en aval pour la séparation du mélange réactionnel quittant le réacteur d'oxyde d'alkylène R1 en un courant partiel a contenant principalement une oléfine en C₂-C₆ et éventuellement d'autres constituants du mélange réactionnel séparés avec celle-ci, et en un courant partiel b contenant de l'eau, de l'oxyde d'alkylène, de l'alkylène glycol et d'autres composants du mélange réactionnel, ainsi qu'éventuellement un solvant organique, et éventuellement un dispositif de séparation B raccordé en aval du dispositif de séparation A, pour la séparation du courant partiel b en un courant partiel c contenant de l'oxyde d'alkylène et éventuellement d'autres constituants du mélange réactionnel séparés avec celui-ci, et en un courant partiel d contenant de l'eau, de l'alkylène glycol et d'autres composants du mélange réactionnel, ainsi qu'éventuellement un solvant organique,
D) la seconde unité partielle comprenant au moins un réacteur d'alkylène glycol R2, ainsi qu'au moins un dispositif de séparation C raccordé en aval et au moins un dispositif de séparation D raccordé en aval à celui-ci, le mélange réactionnel quittant le réacteur d'alkylène glycol R2 étant séparé dans le dispositif de séparation C en un courant partiel e constitué par l'eau contenue dans le mélange réactionnel et éventuellement d'autres constituants du mélange réactionnel séparés avec l'eau, ainsi qu'éventuellement également en un courant partiel f constitué par le solvant organique contenu dans le mélange réactionnel et éventuellement d'autres constituants du mélange réactionnel séparés avec le solvant organique, et en un courant partiel g formé par les alkylène glycols et les autres parties du mélange réactionnel non passées dans le courant partiel e et non dans le courant partiel f, ainsi qu'éventuellement en un courant partiel i contenant des sels et d'autres solides issus du mélange réactionnel, et éventuellement en un courant partiel j contenant des éthers de monoalkylène glycol,
E) le réacteur d'alkylène glycol comprenant au moins une conduite E) pour l'introduction d'oxyde d'alkylène, cette conduite E) pouvant être omise en cas d'introduction du courant partiel b dans le réacteur d'alkylène glycol, et
F) au moins une conduite F) étant prévue, par laquelle au moins une partie du courant partiel b et/ou du courant partiel d issu de la première unité partielle, éventuellement après ajustement du pH, est introduite dans le réacteur d'alkylène glycol R2 de la seconde unité partielle.

11. Unité selon la revendication 10, **caractérisée en ce qu'**une conduite G) est prévue, par laquelle au moins une partie du courant partiel e est transférée dans une unité de purification de l'eau résiduaire, et/ou **en ce qu'**une conduite H) est prévue, par laquelle le courant partiel f, de préférence après purification supplémentaire, est recyclé dans le réacteur d'oxyde d'alkylène, et/ou **en ce qu'**une conduite I) est prévue, par laquelle au moins une partie du courant partiel a est recyclée dans le réacteur d'oxyde d'alkylène, et/ou en ce qu'une conduite J) est prévue, par laquelle une partie du courant partiel e est recyclée dans l'entrée du réacteur d'alkylène glycol, et/ou **en ce qu'**une conduite K) est prévue, par laquelle des sels et d'autres solides contenus dans le mélange réactionnel sont déchargés de l'unité en tant que courant partiel i, et/ou **en ce qu'**une conduite L) est prévue, par laquelle des éthers de monoalkylène glycols sont déchargés de l'unité en tant que courant partiel j.

12. Unité selon la revendication 11, **caractérisée en ce que** des conduites F) et J) sont prévues et **en ce que** des moyens sont prévus, avec lesquels la quantité des courants partiels introduits par les conduites F) et J) dans le réacteur d'alkylène glycol peut être réglée.

13. Unité selon la revendication 10, **caractérisée en ce que** le dispositif de séparation A et le dispositif de séparation B sont présents et sont à chaque fois une colonne de rectification ou un groupe de colonnes de rectification raccordées entre elles, l'oxyde d'alkylène étant de préférence soutiré par la tête de la colonne dans le dispositif de séparation B, ainsi qu'éventuellement également en tant que soutirage latéral, et un produit de fond formant le courant partiel d.

14. Unité selon la revendication 10, **caractérisée en ce que** le réacteur d'oxyde d'alkylène R1 est un réacteur à faisceau de tubes et/ou **en ce que** le réacteur d'alkylène glycol R2 est un réacteur tubulaire.

15. Unité selon la revendication 10, **caractérisée en ce que** le dispositif de séparation C comprend plusieurs colonnes de rectification raccordées en parallèle, dans lesquelles à chaque fois des parties du mélange réactionnel sortant du réacteur d'alkylène glycol sont introduites, et **en ce que** des moyens sont prévus pour utiliser l'énergie thermique issue de la tête d'au moins une colonne de rectification pour le chauffage du fond d'au moins une autre colonne de rectification, ou en ce que le dispositif de séparation C comprend plusieurs évaporateurs raccordés en série, **en ce que** le mélange réactionnel sortant du réacteur d'alkylène glycol est introduit dans le premier évaporateur, et **en ce que** des moyens sont prévus pour utiliser la chaleur de condensation du courant de vapeur sortant d'au moins un évaporateur pour le chauffage d'un autre évaporateur, de préférence de l'évaporateur suivant.

16. Unité selon la revendication 10, **caractérisée en ce que** le dispositif de séparation C comprend au moins une colonne de rectification, dans laquelle 90 à 98 % de l'eau présente est séparée, et **en ce qu'**une autre colonne de rectification est présente, dans laquelle le résidu de l'eau est séparé.

17. Unité selon la revendication 10, **caractérisée en ce que** le dispositif de séparation D comprend plusieurs étapes raccordées les unes après les autres, l'alkylène glycol étant séparé dans la première étape, le dialkylène glycol dans la deuxième étape et le trialkylène glycol et éventuellement d'autres composants de point d'ébullition moyen ou élevé dans une troisième étape éventuellement présente, et des moyens pour le déchargement du produit de fond restant de l'unité étant prévus dans la dernière étape, les étapes du dispositif de séparation D étant de préférence des colonnes de rectification.
